# FASCICULE DE BREVET EUROPEEN

(11) **EP 3 274 362 B1**
(45) Date de publication et mention de la délivrance du brevet: **27.08.2025**
(21) Numéro de dépôt: 16729952.8
(22) Date de dépôt: 24.03.2016
(51) Int. Cl.: C07K 7/08

(54) **NOUVEAU PROCEDE PERMETTANT DE FAVORISER LA NODULATION CHEZ LES PLANTES**
NEUARTIGES VERFAHREN ZUR FÖRDERUNG VON NODULATION IN PFLANZEN
NOVEL METHOD FOR PROMOTING NODULATION IN PLANTS

(30) Priorité: 24.03.2015 FR 1552461
(43) Date de publication de la demande: 31.01.2018
(73) Titulaire: Université Toulouse III-Paul Sabatier, 31062 Toulouse Cedex 9 (FR); Centre National de la Recherche Scientifique, 75794 Paris Cedex 16 (FR)
(72) Inventeur: COMBIER, Jean-Philippe, 31320 Castanet Tolosan (FR); ANDRE, Olivier, 31320 Castanet-Tolosan (FR)
(74) Mandataire: Grosset-Fournier, Chantal Catherine
(86) Numéro de dépôt international: PCT/FR2016/050674
(87) Numéro de publication internationale: WO 2016/151262

(56) Documents cités:
- CN-A- 103 695 432
- FR-A1- 3 012 471
- WANG YOUNING ET AL: "Soybean miR172c Targets the Repressive AP2 Transcription Factor NNC1 to Activate ENOD40 Expression and Regulate Nodule Initiation", PLANT CELL, vol. 26, no. 12, December 2014 (2014-12-01), pages 4782 - 4801, XP002753717
- BARDOU F ET AL: "Dual RNAs in plants", BIOCHIMIE, vol. 93, no. 11, 1 November 2011 (2011-11-01), MASSON, PARIS, FR, pages 1950 - 1954, XP002734738, ISSN: 0300-9084, [retrieved on 20110731], DOI: 10.1016/J.BIOCHI.2011.07.028
- VOINNET: "Origin, Biogenesis, and Activity of Plant MicroRNAs", CELL, vol. 136, no. 4, 20 February 2009 (2009-02-20), CELL PRESS, US, pages 669 - 687, XP008112762, ISSN: 0092-8674, DOI: 10.1016/J.CELL.2009.01.046
- WANG YOUNING ET AL: "MicroRNA167-Directed Regulation of the Auxin Response Factors GmARF8a and GmARF8b Is Required for Soybean Nodulation and Lateral Root Development", July 2015, PLANT PHYSIOLOGY (ROCKVILLE), VOL. 168, NR. 3, PAGE(S) 101-116, ISSN: 0032-0889(print), XP002760677
- COUZIGOU JEAN-MALO ET AL: "miRNA-encoded peptides (miPEPs): A new tool to analyze the roles of miRNAs in plant biology", RNA BIOLOGY, vol. 12, no. 11, November 2015 (2015-11-01), pages 1178 - 1180, XP002753719, ISSN: 1547-1180, DOI: 10.1080/15476286.2015.1094601
- DOMINIQUE LAURESSERGUES ET AL: "Primary transcripts of microRNAs encode regulatory peptides", NATURE, vol. 520, no. 7545, 25 March 2015 (2015-03-25), United Kingdom, pages 90 - 93, XP055225397, ISSN: 0028-0836, DOI: 10.1038/nature14346

## Description

La présente invention concerne l'utilisation de micropeptides (peptides codés par des microARNs ou « miPEPs ») pour favoriser la nodulation chez les plantes.

Chez les plantes, l'acquisition des nutriments à partir du sol est d'une importance majeure et peut constituer une limite à leur développement. Certaines plantes ont ainsi développé la capacité d'établir des associations symbiotiques avec des micro-organismes du sol dans le but d'améliorer l'acquisition des nutriments.

Les plantes légumineuses sont par exemple capables d'établir une symbiose avec des bactéries de la famille *Rhizobiaceae.* La plante hôte et la bactérie vont ainsi se reconnaître par le biais d'un échange de signaux moléculaires. La plante va notamment produire des flavonoïdes qui vont induire l'expression de gènes spécifiques chez la bactérie, conduisant à la production de composés lipochito-oligosaccharidiques, les facteurs Nod, à l'origine de nombreux changements développementaux chez la plante hôte. Les bactéries vont ensuite pénétrer dans les poils des racines des plantes, et induire la formation de nodules. Ce mécanisme de colonisation de la plante par la bactérie est appelé « la nodulation ».

La plante fournit alors aux bactéries à la fois des nutriments et une source d'énergie sous forme d'adénosine triphosphate (ATP) généré par la photosynthèse. En retour, les bactéries fixent l'azote atmosphérique sous forme d'ammonium, fournissant ainsi une importante source d'azote assimilable par la plante.

L'azote est un nutriment dont l'apport s'avère bien souvent déterminant pour la croissance des plantes. De manière générale, plus la fixation de l'azote est élevée chez les plantes, plus les récoltes sont importantes. En conséquence, le taux de nodulation des plantes est bien souvent associé à une récolte plus importante. Une nodulation efficace des plantes permet également de réduire les quantités d'engrais et de fertiliser les sols en composés azotés assimilables par les plantes. De plus, la teneur en protéines est également plus élevée dans les plantes légumineuses par rapport aux autres familles de plantes, en raison de la fixation de l'azote par les bactéries. La teneur élevée en protéines rend ainsi les plantes légumineuses particulièrement importantes à la fois pour la consommation humaine et l'alimentation animale.

L'inoculation des plantes avec des souches spécifiques de bactéries permet de favoriser la nodulation. Néanmoins, bien que différentes techniques soient disponibles, l'inoculation s'avère bien souvent inefficace. Par conséquent, il est nécessaire de fournir un traitement simple pour améliorer la nodulation des plantes.

Les microARNs (miRs) sont des petits ARNs non codants, d'environ 21 nucléotides après maturation, qui contrôlent l'expression de gènes cibles au niveau post-transcriptionnel, en dégradant l'ARNm cible ou en inhibant sa traduction. Les miRs sont notamment rencontrés chez les plantes, et les gènes ciblés par les miRs sont souvent des gènes clés de processus développementaux.

La régulation de l'expression des miRs est très peu connue, mais il a été démontré que celle-ci fait intervenir, à l'instar de la plupart des gènes codants, une ARN polymerase II : cette enzyme produit un transcrit primaire, appelé *« pri-miR »,* qui est ensuite maturé par un complexe protéique contenant notamment les enzymes type Dicer. Cette maturation conduit tout d'abord à la formation d'un précurseur de miR appelé *« pre-miR »,* ayant une structure secondaire en forme tige-boucle contenant le *miR* et sa séquence *miR** complémentaire. Puis le précurseur est maturé, ce qui conduit à la formation d'un ARN double brin plus court contenant le miR et le miR*. Le miR est ensuite pris en charge par le complexe RISC qui clive l'ARNm du gène cible ou bien inhibe sa traduction.

En particulier, le miR172c a été identifié chez les plantes légumineuses telles que la luzerne, le lotier, le soja et le haricot (Lelandais-Brière et al., Genome-wide Medicago truncatula small RNA analysis revealed novel microRNAs and isoforms differentially regulated in roots and nodules, Plant Cell 21 : 2780-2796, 2009 ; Wang et al., Identification and expression analysis of miRNAs from nitrogen-fixing soybean nodules, Biochem Biophys Res Commun 378 : 799-803, 2009 ; Valdes-Lôpez et al., Essential role of MYB transcription factor : PvPHR1 and microRNA : PvmiR399 in phosphorus deficiency signaling in common bean roots, Plant Cell Environ 31 : 1834-1843, 2008 ; De Luis et al., Two microRNAs linked to nodule infection and nitrogen-fixing ability in the legume Lotus japonicus, Plant Physiol 160 : 2137-2154, 2012).

Récemment, il a été montré que le miR172c régule la formation des nodules en réduisant l'expression de son gène cible *NNC1* (Nodule Number Control 1), un facteur de transcription du gène *AP2,* qui réduit l'expression du gène *NOD40,* essentiel dans le processus de nodulation chez le soja (Wang et al., Soybean miR172c targets the repressive AP2 transcription factor NNC1 to activate ENOD40 expression and regulate module initiation, Plant Cell 26 : 4782-4801, 2014). Une autre étude récente a également montré qu'une augmentation de la quantité de miR172c entraîne une augmentation de la nodulation et de la fixation d'azote chez le haricot (Nova-Franco et al., The miR172c-AP2-1 Node as a Key Regulator of the Common Bean - Rhizobia Nitrogen Fixation Symbiosis, Plant Physiol. doi:10.1104/pp.114.255547, March 2015).

Jusqu'à présent, les miRs, et par extension leur transcrit primaire, ont toujours été considérés, de par leur mode d'action particulier, comme des ARNs régulateurs non codants et ne produisant aucun peptide. Or, les Inventeurs ont récemment mis en évidence dans la demande de brevet FR 13/60727 l'existence de micropeptides (ou « miPEPs », *microRNA encoded PEPtides)* capables de moduler l'accumulation des miRs dans les cellules.

Dans ce contexte, la présente invention a pour but de proposer de nouveaux outils efficaces et écologiques pour favoriser la nodulation chez les plantes.

L'un des aspects de l'invention est de proposer une nouvelle utilisation des miPEPs pour favoriser la nodulation entre une plante et une bactérie.

Un autre aspect de l'invention concerne un miPEP permettant de favoriser la nodulation des plantes.

Un autre aspect de l'invention concerne également un nouveau procédé de culture de plantes en symbiose avec une bactérie.

Un autre aspect de l'invention est de proposer une composition de miPEPs permettant de favoriser la nodulation entre une plante et une bactérie.

L'invention a également pour objet une plante transgénique et des parties de plantes transgéniques, leur procédé de production, ainsi que des organes, cellules et semences de plantes transgéniques, des plantes modifiées écologiquement, et des inocula de bactéries.

En résumé de ce qui suit, l'invention est telle que définie dans les revendications 1 à 15.

L'invention concerne donc l'utilisation d'un peptide pour favoriser la nodulation entre une plante et une bactérie, ledit peptide étant introduit dans la plante, ledit peptide ayant une séquence d'acides aminés comprenant ou consistant en une séquence identique à celle d'un miPEP naturellement présent dans ladite plante,
ledit miPEP naturellement présent dans ladite plante étant un peptide de 3 à 100 acides, notamment de 4 à 100 acides aminés, dont la séquence est codée par un cadre ouvert de lecture situé sur le transcrit primaire d'un miR, ledit miPEP étant capable de moduler l'accumulation dudit miR dans ladite plante, lequel miR régule l'expression d'au moins un gène impliqué dans la nodulation chez ladite plante.

De manière surprenante et inattendue, les Inventeurs ont constaté que l'utilisation de peptides dont la séquence comprend ou consiste en une séquence identique à celle de miPEPs codés sur les transcrits primaires de miRs, permet de favoriser la nodulation entre une plante et une bactérie.

Dans l'invention, les termes *« microARN », « microARN non codant »* et *« miR »* sont équivalents et peuvent être utilisés l'un pour l'autre. Ils définissent des petites molécules d'ARN d'environ 21 nucléotides, qui ne sont pas traduites et ne conduisent pas à un peptide ou une protéine. Cependant, sous cette forme mature, les miRs assurent une fonction de régulation de certains gènes *via* des mécanismes post-transcriptionnels, comme par exemple par l'intermédiaire du complexe RISC.

Le *« transcrit primaire de miR »* (ou *« pri-miR* ») correspond lui à la molécule d'ARN directement obtenue à partir de la transcription de la molécule d'ADN. Généralement, ce transcrit primaire subit une ou plusieurs modifications post-transcriptionnelles, qui entraînent par exemple une structure particulière de l'ARN ou un clivage de certaines parties de l'ARN par des phénomènes d'épissage, et qui conduisent à la forme précurseur du miR ou (« *pre-miR* »), puis à la forme mature du miR

Les termes *« micropeptides »* et *« miPEPs »* (*microRNA encoded PEPtides*) sont équivalents et peuvent être utilisés l'un pour l'autre. Ils définissent un peptide qui est codé par un cadre ouvert de lecture présent sur le transcrit primaire d'un miR, et qui est capable de moduler l'accumulation dudit miR. Les miPEPs au sens de la présente invention ne doivent être entendus comme étant nécessairement des peptides de petite taille, dans la mesure où « micro » ne correspond pas à la taille du peptide.

Comme indiqué dans la demande de brevet FR 13/60727, dont le contenu doit être considéré comme faisant partie de la présente demande, les miPEPs sont des peptides :
- de 4 à 100 acides aminés, de préférence de 4 à 60 acides aminés, notamment de 4 à 59 acides aminés,
- codés par un cadre ouvert de lecture contenu dans le transcrit primaire d'un miR, de préférence dans la partie 5' du transcrit primaire dudit miR, et
- capables de moduler l'accumulation dudit miR dans une cellule eucaryote.

Les termes « *cadre ouvert de lecture »* ou « *ORF » (open reading frame*) sont équivalents et peuvent être utilisés l'un pour l'autre. Ils correspondent à une séquence de nucléotides dans une molécule d'ADN ou d'ARN pouvant potentiellement coder un peptide ou une protéine: ledit cadre ouvert de lecture débute par un codon start (le codon start codant généralement une méthionine), suivi d'une série de codons (chaque codon codant un acide aminé), et se termine par un codon stop (le codon stop n'étant pas traduit).

Dans l'invention, les ORFs peuvent être dénommés de manière spécifique *« miORFs »* lorsque ceux-ci sont présents sur les transcrits primaires de miR.

Les miORFs tels que définis dans l'invention peuvent avoir une taille de 15 à 303 nucléotides. Un acide aminé étant codé par un codon de 3 nucléotides, les miORFs de 15 à 303 nucléotides codent des miPEPS de 4 à 100 acides aminés.

En particulier, les miORFs ont une taille de :
15, 18, 21, 24, 27, 30, 33, 36, 39, 42, 45, 47, 51, 54, 57, 60, 63, 66, 69, 72, 75, 78, 81, 84, 87, 90, 93, 96, 99, 102, 105, 108, 111, 114, 117, 120, 123, 126, 129, 132, 135, 138, 141, 144, 147, 150, 153, 156, 159, 162, 165, 168, 171, 174, 177, 180, 183, 186, 189, 192, 195, 198, 201, 204, 207, 210, 213, 216, 219, 222, 225, 228, 231, 234, 237, 240, 243, 246, 249, 252, 255, 258, 261, 264, 267, 270, 273, 276, 279, 282, 285, 288, 291, 294, 297, 300 ou 303 nucléotides, et codent respectivement des miPEPs ayant une taille de :
4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ou 100 acides aminés.

Un miPEP peut également avoir une taille de 3 à 100 acides aminés.

Compte tenu de la dégénérescence du code génétique, un même miPEP peut être codé par plusieurs séquences nucléotidiques. De telles séquences nucléotidiques, différentes entre elles d'au moins un nucléotide mais codant un même peptide, sont appelées *« séquences dégénérées ».*

Dans l'invention, le terme *« plante* » fait référence de manière générale à tout ou partie d'une plante quel que soit son stade de développement (y compris la plante sous forme de graine ou de jeune pousse), à un ou plusieurs organes de la plante (comme par exemple les feuilles, les racines, la tige, les fleurs), à une ou plusieurs cellules de la plante, ou encore à un amas de cellules de la plante.

L'expression *« nodulation »* fait référence à la colonisation d'une plante par une bactérie symbiote aboutissant à la formation de nodules sur les racines.

De manière non limitative, les paramètres permettant de déterminer et quantifier la nodulation entre une plante et une bactérie peuvent être notamment :
- la taille et le nombre des nodules,
- la teneur en azote dans tout ou partie de la plante,
- ou de manière indirecte, le développement de certaines parties de la plante, telles que les gousses ou les racines.

Les *"nodules''* peuvent également être appelées *"nodosités".* Il s'agit de boursouflures se formant sur les racines des plantes sous l'action de bactéries. Leur taille et leur nombre peuvent être mesurés à l'œil nu ou par microscope, à l'aide de techniques connues de l'homme du métier. Leur taille est mesurée après une culture de longue durée (supérieure à 30 jours) en mesurant la taille de nodules sur des photographies, par exemple à l'aide de logiciels type ImageJ.

La *"teneur en azote''* correspond à la concentration d'azote dans la plante ou dans une partie de la plante. Par exemple, il peut s'agir de la concentration d'azote dans les parties aériennes en g d'azote par kg de poids sec de feuilles. Cette concentration d'azote peut être déterminée par des techniques connues de l'homme du métier. Par exemple, une méthode consiste à brûler un échantillon de masse connue dans une température élevée (environ 900° C) en présence d'oxygène, conduisant à la libération de dioxyde de carbone, eau et azote. Les gaz sont ensuite passés sur des colonnes particulières (comme une solution aqueuse d'hydroxyde de potassium) qui absorbent le dioxyde de carbone et eau. Une colonne contenant un détecteur de conductivité thermique à la fin est ensuite utilisé pour séparer l'azote de tout dioxyde de carbone résiduel et de l'eau et la teneur en azote résiduelle est quantifiée.

Le *"développement de certaines parties de la plante"* correspond à la croissance ou à la maturation de certaines parties de la plante. Le développement de certaines parties de la plante peut par exemple être déterminé en mesurant la vitesse de croissance ou la taille ou le poids de certaines parties de la plante. Ce paramètre peut être aisément mesuré en effectuant par exemple des mesures de poids sec des racines, des gousses ou des parties aériennes.

Par ailleurs, dans l'invention, l'expression *« favoriser la* nodulation», ou *« améliorer la nodulation»,* indique :
- soit une accélération de la nodulation (comme par exemple une formation plus rapide des nodules chez une plante donnée par rapport à une plante de référence),
- soit à un accroissement de la nodulation (comme par exemple un nombre plus important ou une taille plus importante des nodules chez une plante donnée par rapport à une plante de référence),
- soit à une accélération et un accroissement de la nodulation.

Il est important de noter que l'utilisation selon l'invention possède l'avantage d'être écologique, car le miPEP est un peptide qui est présent naturellement chez la plante.

L'invention concerne également l'utilisation d'un miPEP introduit par voie exogène dans une plante pour favoriser la nodulation entre ladite plante et une bactérie,
ledit miPEP introduit par voie exogène étant un peptide comprenant, ou consistant en, une séquence identique à celle d'un miPEP naturellement présent dans ladite plante,
lequel miPEP naturellement présent est un peptide de 3 à 100 acides aminés, notamment de 4 à 100 acides aminés, dont la séquence est codée par un cadre ouvert de lecture situé en 5' sur le transcrit primaire d'un miR,
ledit miPEP étant capable de moduler l'accumulation dudit miR dans ladite plante, lequel miR régule l'expression d'au moins un gène impliqué dans la nodulation chez ladite plante, la somme de la quantité dudit miPEP introduit par voie exogène et de celle dudit miPEP naturellement présent étant strictement supérieure à la quantité dudit miPEP naturellement présent.

Dans l'invention, l'expression « *miPEP introduit par voie exogène »* fait référence à un miPEP introduit artificiellement dans la plante que celui-ci existe ou non naturellement dans la plante.

L'introduction d'un miPEP par voie exogène dans la plante implique donc une étape technique, laquelle étape n'est pas un phénomène naturel et ne correspond ni à un croisement, ni à une sélection.

Le miPEP introduit par voie exogène peut être soit un peptide produit hors de la plante (comme par exemple un peptide isolé et/ou purifié, un peptide synthétique ou un peptide recombinant), soit un peptide produit dans la plante suite à l'introduction non naturelle d'un acide nucléique codant ledit miPEP dans ladite plante.

Si le miPEP existe naturellement chez la plante, il s'agit d'un « *miPEP d'origine endogène ».* Si le miPEP n'existe pas naturellement chez la plante, il s'agit d'un *« miPEP d'origine exogène ».*

Lorsqu'il est introduit dans la plante un « *miPEP d'origine exogène »,* il est alors nécessaire d'introduire également le miR correspondant et son transcrit primaire.

La plante dans laquelle le miPEP n'a pas été introduit possède une quantité basale dudit miPEP, qui correspond à celle dudit miPEP naturellement présent. L'utilisation d'un miPEP comprenant, ou consistant en, une séquence identique à celle dudit miPEP entraîne une augmentation de la quantité totale de miPEP, ce qui module l'accumulation du miR dont le transcrit primaire contient la séquence codant ledit miPEP.

Par ailleurs, le miPEP introduit se retrouve dans la plante et son introduction n'a pas d'impact sur la stabilité de celui-ci.

Dans l'invention, par « *accumulation »,* on entend la production d'une molécule, telle qu'un miR ou un miPEP, dans la cellule.

Ainsi, la *« modulation de l'accumulation »* d'une molécule dans une cellule correspond à une modification de la quantité de cette molécule dans la cellule.

Par ailleurs, l'effet d'un miPEP peut être observé via la modulation de l'accumulation du miR, mais également à travers la modulation de l'accumulation du pri-miR ou du pre-miR correspondant.

Dans un mode de réalisation, l'invention concerne l'utilisation telle que définie ci-dessus, dans lequel la modulation de l'accumulation dudit miR est une diminution ou une augmentation de l'accumulation dudit miR, en particulier une augmentation.

Une « *diminution de l'accumulation du miR»* correspond à une baisse de la quantité de ladite molécule dans la cellule.

A l'inverse, une *« augmentation de l'accumulation du miR»* correspond à une hausse de la quantité de ladite molécule dans la cellule.

Dans un mode de réalisation, l'invention concerne l'utilisation telle que définie ci-dessus dans laquelle ledit gène impliqué dans la nodulation code un facteur de transcription de la famille AP2.

Dans un mode de réalisation, l'invention concerne l'utilisation telle que définie ci-dessus, dans laquelle ledit gène impliqué dans la nodulation est le facteur de transcription *NNC1.*

Dans un mode de réalisation, l'invention concerne l'utilisation telle que définie ci-dessus, dans laquelle ledit gène impliqué dans la nodulation est le gène *NSP1.*

Dans un mode de réalisation, l'invention concerne l'utilisation telle que définie ci-dessus, dans laquelle ledit gène impliqué dans la nodulation est le gène *NIN.*

Dans un mode de réalisation, l'invention concerne l'utilisation telle que définie ci-dessus, dans laquelle ledit gène impliqué dans la nodulation est le gène *ENOD40-1.*

Dans un mode de réalisation, l'invention concerne l'utilisation telle que définie ci-dessus, dans laquelle ledit gène impliqué dans la nodulation est le gène *Hb2.*

Dans un mode de réalisation, l'invention concerne l'utilisation telle que définie ci-dessus, dans laquelle ledit gène impliqué dans la nodulation est le gène *nifH.*

En particulier, l'invention concerne l'utilisation telle que définie ci-dessus, dans laquelle ledit miR172c possède une séquence nucléotidique présentant au moins 80% d'identité, de préférence au moins 90 % d'identité, avec la séquence nucléotidique SEQ ID NO : 1.

Dans un mode de réalisation, l'invention concerne l'utilisation telle que définie ci-dessus, dans laquelle ledit miARN est le miR172c, en particulier, dans laquelle ledit miR172c possède une séquence nucléotidique comprenant ou consistant en SEQ ID NO : 1.

En particulier, l'invention concerne l'utilisation telle que définie ci-dessus, dans laquelle ledit miPEP172c possède une séquence d'acides aminés présentant au moins 80% d'identité, de préférence au moins 90 % d'identité, avec la séquence d'acides aminés SEQ ID NO : 2.

Dans un mode de réalisation, l'invention concerne l'utilisation telle que définie ci-dessus, dans laquelle ledit miPEP est le miPEP172c, en particulier, dans laquelle ledit miPEP172c possède une séquence d'acides aminés comprenant ou consistant en SEQ ID NO : 2.

En particulier, est décrite l'utilisation telle que définie ci-dessus, dans laquelle ledit miR167c possède une séquence nucléotidique présentant au moins 80% d'identité, de préférence au moins 90 % d'identité, avec la séquence nucléotidique SEQ ID NO : 6.

Dans un mode de réalisation, est décrite l'utilisation telle que définie ci-dessus, dans laquelle ledit miARN est le miR167c, en particulier, dans laquelle ledit miR167c possède une séquence nucléotidique comprenant ou consistant en SEQ ID NO : 6.

En particulier, est décrite l'utilisation telle que définie ci-dessus, dans laquelle ledit miPEP167c possède une séquence d'acides aminés présentant au moins 80% d'identité, de préférence au moins 90 % d'identité, avec la séquence d'acides aminés SEQ ID NO : 7.

Dans un mode de réalisation, est décrite l'utilisation telle que définie ci-dessus, dans laquelle ledit miPEP est le miPEP167c, en particulier, dans laquelle ledit miPEP167c possède une séquence d'acides aminés comprenant ou consistant en SEQ ID NO : 7.

Dans un mode de réalisation, l'invention concerne l'utilisation telle que définie ci-dessus, dans laquelle ladite plante est une plante légumineuse, telle que le Lotus *(Lotus sp.)* le soja *(Glycine max),* l'arachide *(Arachis hypogaea),* le haricot *(Phaseolus vulgaris),* le pois *(Pisum sativum),* la lentille (*Lens culinaris),* le pois chiche *(Cicer arietinum),* la fève et la féverolle *(Vicia faba*), les vesces *(Vicia* sp.), les gesses *(Lathyrus* sp.), la luzerne *(Medicago* sp.), le trèfle *(Trifolium* sp.), le lupin *(Lupinus* sp.), le haricot mungo (*Vigna radiata),* la réglisse *(Glycyrrhiza glabra),* le palissandre *(Dalbergia),* le lotier corniculé *(Lotus corniculatus),* le sainfoin *(Onobrychis viciifolia*), le rooibos *(Aspalathus linearis),* le fenugrec *(Trigonella foenum-graecum*).

Dans un mode de réalisation, l'invention concerne l'utilisation telle que définie ci-dessus, dans laquelle ladite plante est la betterave sucrière (*Beta vulgaris*).

Dans un mode de réalisation, l'invention concerne l'utilisation telle que définie ci-dessus, dans laquelle ladite plante est choisie parmi *Medicago truncatula, M. sativa* et *Glycine max.*

Dans un mode de réalisation, l'invention concerne l'utilisation telle que définie ci-dessus, dans laquelle ladite plante est *Glycine max.*

Dans un mode de réalisation, l'invention concerne l'utilisation telle que définie ci-dessus, dans laquelle ladite bactérie est une bactérie de la famille des *Rhizobiaceae.*

Dans un mode de réalisation particulier, l'invention concerne l'utilisation telle que définie ci-dessus, dans laquelle ladite bactérie est choisie parmi les genres *Rhizobium, Sinorhizobium, Mesorhizobium, Bradyrhizobium* ou *Azorhizobium.*

Dans l'invention, ladite bactérie peut également être choisie parmi :
- *Rhizobium alamii, Rhizobium alkalisoli, Rhizobium cellulosilyticum, Rhizobium daejeonense, Rhizobium endophyticum, Rhizobium etli, Rhizobium galegae, Rhizobium gallicum, Rhizobium giardinii, Rhizobium hainanense, Rhizobium herbae, Rhizobium huautlense, Rhizobium indigoferae, Rhizobium leguminosarum, Rhizobium loessense, Rhizobium lusitanum, Rhizobium mesosinicum, Rhizobium miluonense, Rhizobium mongolense, Rhizobium multihospitium, Rhizobium oryzae, Rhizobium phaseoli, Rhizobium pisi, Rhizobium tibeticum, Rhizobium sullae, Rhizobium tropici, Rhizobium tubonense, Rhizobium undicola, Rhizobium vignae, Rhizobium yanglingense,*
- *Mesorhizobium albiziae, Mesorhizobium alhagi, Mesorhizobium amorphae, Mesorhizobium australicum, Mesorhizobium camelthorni, Mesorhizobium caraganae, Mesorhizobium chacoense, Mesorhizobium cicero, Mesorhizobium gobiense, Mesorhizobium huakuii, Mesorhizobium loti, Mesorhizobium mediterraneum, Mesorhizobium metallidurans, Mesorhizobium opportunistum, Mesorhizobium plurifarium, Mesorhizobium robiniae, Mesorhizobium shangrilense, Mesorhizobium septentrionale, Mesorhizobium tarimense, Mesorhizobium temperatum, Mesorhizobium tianshanense*
- *Ensifer abri, Sinorhizobium americanum, Ensifer arboris, Ensifer fredii, Ensifer garamanticus, Ensifer indiaense, Ensifer kostiensis, Ensifer kummerowiae, Ensifer medicae, Ensifer meliloti, Ensifer mexicanus, Sinorhizobium morelense, Ensifer adhaerens, Ensifer numidicus, Ensifer saheli, Ensifer sojae, Ensifer terangae,*
- *Bradyrhizobium canariense, Bradyrhizobium denitrificans, Bradyrhizobium elkanii, Bradyrhizobium iriomotense, Bradyrhizobium japonicum, Bradyrhizobium jicamae, Bradyrhizobium liaoningense, Bradyrhizobium pachyrhizi, Bradyrhizobium yuanmingense*
- *Burkholderia caribensis, Burkholderia cepacia, Burkholderia mimosarum, Burkholderia nodosa, Burkholderia phymatum, Burkholderia sabiae, Burkholderia tuberum*
- *Phyllobacterium trifolii, Phyllobacterium ifriqiyense, Phyllobacterium leguminum*
- *Microvirga lupine, Microvirga lotononidis, Microvirga zambiensis*
- *Azorhizobium caulinodans, Azorhizobium doebereinerae*
- *Ochrobactrum cytisi, Ochrobactrum lupini*
- *Methylobacterium nodulans,*
- *Cupriavidus taiwanensis,*
- *Devosia neptuniae,*
- *Shinella kummerowiae.*

Dans un mode de réalisation, l'invention concerne l'utilisation telle que définie ci-dessus, dans laquelle ladite bactérie est une bactérie choisie parmi *Rhizobium leguminosarum, Rhizobium etli, Rhizobium tropici, Rhizobium galegae, Sinorhizobium* sp. NGR234, *Sinorhizobium meliloti, Sinorhizobium fredii, Sinorhizobium saheli, Sinorhizobium teranga, Mesorhizobium ciceri, Mesorhizobium huakuii, Mesorhizobium loti, Bradyrhizobium elkanii, Bradyrhizobium japonicum, Bradyrhizobium lupini, Bradyrhizobium* sp. "cowpea" et *Azorhizobium caulinodans.*

En particulier, la bactérie *Rhizobium elti* permet la nodulation du haricot.

En particulier, la bactérie *Rhizobium leguminosarum* permet la nodulation du pois, de la Luzerne, de la betterave sucrière, de la vesce, de la lentille.

En particulier, la bactérie *Rhizobium pisi* permet la nodulation du pois, du haricot, de la vesce.

En particulier, la bactérie *Rhizobium phaseoli* permet la nodulation du haricot sec, du haricot.

En particulier, la bactérie *Rhizobium fabae* permet la nodulation de la fève.

En particulier, la bactérie *Rhizobium ciceri* permet la nodulation du pois chiche.

En particulier, la bactérie *Rhizobium trifoli* permet la nodulation du trèfle.

En particulier, la bactérie *Sinorhizobium meliloti* permet la nodulation de la Luzerne, du fenugrec ou du mélilot.

En particulier, la bactérie *Sinorhizobium fredii* permet la nodulation du soja.

En particulier, la bactérie *Mesorhizobium loti* permet la nodulation du lotus, du trèfle, du lupin, ou du pois chiche.

En particulier, la bactérie *Bradyrhizobium japonicum* permet la nodulation du soja.

En particulier, la bactérie *Bradyrhizobium* sp. *Vigna* permet la nodulation de l'arachide.

En particulier, la bactérie *Bradyrhizobium* sp. *Arachis* permet la nodulation de l'arachide.

Dans un mode de réalisation, l'invention concerne l'utilisation telle que définie ci-dessus, pour favoriser la nodulation entre une plante *Glycine max* et une bactérie *Bradirhizobium japonicum,* dans laquelle le miPEP172c est introduit par voie exogène dans ladite plante *Glycine max,* ledit miPEP172c étant également naturellement présent dans ladite plante *Glycine max,*
ledit miPEP172c introduit par voie exogène étant un peptide dont la séquence comprend ou consiste en une séquence identique à celle dudit miPEP172c naturellement présent, ladite séquence du miPEP172c naturellement présent étant codée par un cadre ouvert de lecture situé en 5' sur le transcrit primaire du miR172c, lequel miR172c régule l'expression d'au moins un gène impliqué dans la nodulation chez *Glycine max,*
la somme de la quantité dudit miPEP172c introduit par voie exogène et de celle dudit miPEP172c naturellement présent étant strictement supérieure à la quantité dudit miPEP172c naturellement présent chez ladite plante *Glycine max.*

Dans un mode de réalisation, l'invention concerne l'utilisation d'un miPEP pour favoriser la nodulation entre une plante *Glycine max* et une bactérie *Bradirhizobium japonicum,* dans laquelle le miPEP172c est introduit par voie exogène dans ladite plante *Glycine max,* ledit miPEP172c étant également naturellement présent dans ladite plante *Glycine max,*
ledit miPEP172c introduit par voie exogène étant un peptide dont la séquence présente au moins 80% d'identité, de préférence au moins 90% d'identité avec la séquence SEQ ID NO : 2, ladite séquence du miPEP172c naturellement présent étant codée par un cadre ouvert de lecture situé en 5' sur le transcrit primaire du miR172c, lequel miR172c régule l'expression d'au moins un gène impliqué dans la nodulation chez *Glycine max,*
la somme de la quantité dudit miPEP172c introduit par voie exogène et de celle dudit miPEP172c naturellement présent étant strictement supérieure à la quantité dudit miPEP172c naturellement présent chez ladite plante *Glycine max.*

Dans un mode de réalisation, l'invention concerne l'utilisation d'un miPEP pour favoriser la nodulation entre une plante *Glycine max* et une bactérie *Bradirhizobium japonicum,* dans laquelle le miPEP172c est introduit par voie exogène dans ladite plante *Glycine max,* ledit miPEP172c étant également naturellement présent dans ladite plante *Glycine max,*
ledit miPEP172c introduit par voie exogène étant un peptide dont la séquence comprend ou consiste en SEQ ID NO : 2, ladite séquence du miPEP172c naturellement présent étant codée par un cadre ouvert de lecture situé en 5' sur le transcrit primaire du miR172c, lequel miR172c régule l'expression d'au moins un gène impliqué dans la nodulation chez *Glycine max,*
la somme de la quantité dudit miPEP172c introduit par voie exogène et de celle dudit miPEP172c naturellement présent étant strictement supérieure à la quantité dudit miPEP172c naturellement présent chez ladite plante *Glycine max.*

Dans un mode de réalisation, l'invention concerne l'utilisation d'un miPEP pour favoriser la nodulation entre une plante *Glycine max* et une bactérie *Bradirhizobium japonicum,* dans laquelle le miPEP172c est introduit par voie exogène dans ladite plante *Glycine max,* ledit miPEP172c étant également naturellement présent dans ladite plante *Glycine max,*
ledit miPEP172c introduit par voie exogène étant un peptide dont la séquence comprend ou consiste en une séquence identique à celle dudit miPEP172c naturellement présent, ladite séquence du miPEP172c naturellement présent étant codée par un cadre ouvert de lecture situé en 5' sur le transcrit primaire du miR172c, lequel miR172c régule l'expression d'au moins un gène impliqué dans la nodulation chez *Glycine max,*
lequel miR172c comprend ou consiste en SEQ ID NO : 1,
la somme de la quantité dudit miPEP172c introduit par voie exogène et de celle dudit miPEP172c naturellement présent étant strictement supérieure à la quantité dudit miPEP172c naturellement présent chez ladite plante *Glycine max.*

Dans un mode de réalisation, est décrite l'utilisation telle que définie ci-dessus, pour favoriser la nodulation entre une plante *Glycine max* et une bactérie *Bradirhizobium japonicum,* dans laquelle le miPEP167c est introduit par voie exogène dans ladite plante *Glycine max,* ledit miPEP167c étant également naturellement présent dans ladite plante *Glycine max,*
ledit miPEP167c introduit par voie exogène étant un peptide dont la séquence comprend ou consiste en une séquence identique à celle dudit miPEP167c naturellement présent, ladite séquence du miPEP167c naturellement présent étant codée par un cadre ouvert de lecture situé en 5' sur le transcrit primaire du miR167c, lequel miR167c régule l'expression d'au moins un gène impliqué dans la nodulation chez *Glycine max,*
la somme de la quantité dudit miPEP167c introduit par voie exogène et de celle dudit miPEP167c naturellement présent étant strictement supérieure à la quantité dudit miPEP167c naturellement présent chez ladite plante *Glycine max.*

Dans un mode de réalisation, est décrite l'utilisation d'un miPEP pour favoriser la nodulation entre une plante *Glycine max* et une bactérie *Bradirhizobium japonicum,* dans laquelle le miPEP167c est introduit par voie exogène dans ladite plante *Glycine max,* ledit miPEP167c étant également naturellement présent dans ladite plante *Glycine max,*
ledit miPEP167c introduit par voie exogène étant un peptide dont la séquence présente au moins 80% d'identité, de préférence au moins 90% d'identité avec la séquence SEQ ID NO : 7, ladite séquence du miPEP167c naturellement présent étant codée par un cadre ouvert de lecture situé en 5' sur le transcrit primaire du miR167c, lequel miR167c régule l'expression d'au moins un gène impliqué dans la nodulation chez *Glycine max,*
la somme de la quantité dudit miPEP167c introduit par voie exogène et de celle dudit miPEP167c naturellement présent étant strictement supérieure à la quantité dudit miPEP167c naturellement présent chez ladite plante *Glycine max.*

Dans un mode de réalisation, est décrite l'utilisation d'un miPEP pour favoriser la nodulation entre une plante *Glycine max* et une bactérie *Bradirhizobium japonicum,* dans laquelle le miPEP167c est introduit par voie exogène dans ladite plante *Glycine max,* ledit miPEP167c étant également naturellement présent dans ladite plante *Glycine max,*
ledit miPEP167c introduit par voie exogène étant un peptide dont la séquence comprend ou consiste en SEQ ID NO : 7, ladite séquence du miPEP 167c naturellement présent étant codée par un cadre ouvert de lecture situé en 5' sur le transcrit primaire du miR167c, lequel miR167c régule l'expression d'au moins un gène impliqué dans la nodulation chez *Glycine max,*
la somme de la quantité dudit miPEP167c introduit par voie exogène et de celle dudit miPEP167c naturellement présent étant strictement supérieure à la quantité dudit miPEP167c naturellement présent chez ladite plante *Glycine max.*

Dans un mode de réalisation, est décrite l'utilisation d'un miPEP pour favoriser la nodulation entre une plante *Glycine max* et une bactérie *Bradirhizobium japonicum,* dans laquelle le miPEP167c est introduit par voie exogène dans ladite plante *Glycine max,* ledit miPEP167c étant également naturellement présent dans ladite plante *Glycine max,*
ledit miPEP167c introduit par voie exogène étant un peptide dont la séquence comprend ou consiste en une séquence identique à celle dudit miPEP167c naturellement présent, ladite séquence du miPEP167c naturellement présent étant codée par un cadre ouvert de lecture situé en 5' sur le transcrit primaire du miR167c, lequel miR167c régule l'expression d'au moins un gène impliqué dans la nodulation chez *Glycine max,*
lequel miR167c comprend ou consiste en SEQ ID NO : 6,
la somme de la quantité dudit miPEP167c introduit par voie exogène et de celle dudit miPEP167c naturellement présent étant strictement supérieure à la quantité dudit miPEP167c naturellement présent chez ladite plante *Glycine max.*

Dans un mode de réalisation, l'invention concerne l'utilisation telle que définie ci-dessus, dans laquelle ledit miPEP est introduit par voie externe dans la plante, de préférence par arrosage, par pulvérisation ou par l'ajout d'un engrais, d'un terreau, d'un substrat de culture ou à l'aide d'un support en contact avec la plante.

Dans un mode de réalisation, l'invention concerne l'utilisation telle que définie ci-dessus, dans laquelle ledit miPEP est introduit par voie externe dans une graine ou une semence, de préférence par arrosage, par pulvérisation ou par l'ajout d'un engrais, d'un terreau, d'un substrat de culture ou à l'aide d'un support en contact avec la graine ou la semence.

Dans un mode de réalisation, l'invention concerne l'utilisation telle que définie ci-dessus, dans laquelle ledit miPEP est utilisé pour traiter la plante sous forme de graine.

Dans un mode de réalisation, l'invention concerne l'utilisation telle que définie ci-dessus, dans laquelle ledit miPEP est introduit par arrosage, notamment par pulvérisation.

Dans un mode de réalisation, l'invention concerne l'utilisation telle que définie ci-dessus, dans laquelle ledit miPEP est introduit par arrosage et par l'ajout d'un engrais.

Dans un mode de réalisation, l'invention concerne l'utilisation telle que définie ci-dessus, dans laquelle ledit miPEP est introduit par l'ajout d'un engrais.

Dans un mode de réalisation, l'invention concerne l'utilisation telle que définie ci-dessus, dans laquelle ledit miPEP est introduit, par arrosage, et par l'ajout d'un engrais.

Dans un mode de réalisation, l'invention concerne l'utilisation telle que définie ci-dessus, dans laquelle ledit miPEP est introduit par l'ajout de granulés.

Les Inventeurs ont en effet constaté de manière inattendue qu'il est possible d'appliquer directement une composition comprenant un miPEP sur la plante pour moduler l'accumulation du miR correspondant dans la plante, ce qui indique que le miPEP est capté par la plante.

Dans un mode de réalisation, l'invention concerne l'utilisation telle que définie ci-dessus, dans laquelle la plante est traitée avec une composition comprenant 10⁻⁹ M à 10⁻⁴ M dudit miPEP, notamment 10⁻⁹ M, 10⁻⁸ M, 10⁻⁷ M, 10⁻⁶ M, 10⁻⁵ M ou 10⁻⁴ M dudit miPEP.

De préférence, les compositions ont une concentration de 10⁻⁸ M à 10⁻⁵ M pour une application par arrosage ou par pulvérisation sur la plante.

De manière complémentaire, des compositions plus ou moins concentrées peuvent être envisagées pour traiter la plante avec le miPEP. Par exemple, et de manière non limitative, des compositions plus concentrées comprenant 10⁻¹ M à 10⁻³ M, notamment 10⁻² M de miPEP, peuvent être utilisées dans le cas où le miPEP introduit par voie exogène est administré à la plante par épandage.

Les propriétés de solubilité des miPEPs sont déterminées notamment par leur composition en acides aminés. Les miPEPs hydrophiles peuvent être solubilisés et conditionnés dans des solutions aqueuses, telles que l'eau. Les miPEPs hydrophobes peuvent être solubilisés et conditionnés dans des solvants, tels que les solvants organiques.

Pour un traitement des plantes par les miPEPs, les solvants organiques sont des solvants non toxiques pour les plantes en faibles quantités, c'est-à-dire qu'ils n'ont pas d'effet délétères sur le développement de la plante. De manière non limitative, les solvants organiques peuvent être choisis parmi l'acétonitrile et l'acide acétique.

Les miPEPs peuvent également être solubilisés et conditionnés dans des mélanges de solvants organiques, comme par exemple un mélange d'acétonitrile et d'acide acétique. En particulier, les miPEPs peuvent être solubilisés dans une solution comprenant 50 % d'acétonitrile, 10% d'acide acétique et 40% d'eau (volume/volume/volume).

En particulier, le miPEP172c est solubilisé dans une solution comprenant 40% eau, 50% acétonitrile, 10% acide acétique (volume/volume/volume) ou dans de l'eau.

En particulier, le miPEP167c est solubilisé dans l'eau.

Dans un mode de réalisation, l'invention concerne l'utilisation telle que définie ci-dessus, dans laquelle ledit miPEP est introduit dans la plante par le biais d'un acide nucléique codant ledit miPEP, ledit acide nucléique étant introduit dans la plante.

Dans un mode de réalisation, l'invention concerne l'utilisation telle que définie ci-dessus, dans laquelle le nombre de nodules est augmenté chez la plante dans laquelle a été introduit ledit miPEP par rapport au nombre de nodules d'une plante identique et de même âge dans laquelle aucun miPEP n'a été introduit, ou bien par rapport au nombre de nodules d'une plante identique et de même âge dans laquelle ledit miPEP n'a pas été introduit.

Dans un mode de réalisation, l'invention concerne l'utilisation telle que définie ci-dessus, dans laquelle la concentration d'azote est augmentée dans les parties aériennes de la plante dans laquelle a été introduit ledit miPEP par rapport à la concentration d'azote dans les parties aériennes d'une plante identique et de même âge dans laquelle aucun miPEP n'a été introduit, ou bien par rapport à la concentration d'azote dans les parties aériennes d'une plante identique et de même âge dans laquelle ledit miPEP n'a pas été introduit.

Dans un mode de réalisation, l'invention concerne l'utilisation telle que définie ci-dessus, dans laquelle le poids des gousses est augmenté chez la plante dans laquelle a été introduit ledit miPEP par rapport au poids des gousses d'une plante identique et de même âge dans laquelle aucun miPEP n'a été introduit, ou bien par rapport au poids des gousses d'une plante identique et de même âge dans laquelle ledit miPEP n'a pas été introduit.

L'augmentation des paramètres permettant de déterminer et de quantifier la nodulation chez la plante dans laquelle a été introduit le miPEP (tels que le nombre de nodules, la concentration d'azote ou encore le poids des gousses) est de préférence mis en évidence par comparaison avec une plante identique (c'est-à-dire une plante de la même espèce et/ou variété), de même âge et cultivée dans les mêmes conditions mais dans laquelle aucun miPEP n'a été introduit.

L'invention concerne également l'utilisation d'un miPEP introduit par voie exogène dans une plante pour favoriser la nodulation entre ladite plante et une bactérie,
ledit miPEP étant codé par le transcrit primaire, introduit artificiellement dans la plante, d'un miR,
ledit transcrit primaire, ledit miR et ledit miPEP étant absents naturellement chez la plante, ledit miPEP étant capable de moduler l'accumulation dudit miR dans ladite plante, lequel miR régule l'expression d'au moins un gène impliqué dans la nodulation chez ladite plante. Dans un mode de réalisation particulier, ledit transcrit primaire du miR, le miR et ledit miPEP sont introduits dans la plante à l'aide d'un vecteur.

Dans un autre aspect, l'invention concerne un procédé pour favoriser la nodulation entre une plante et une bactérie, comprenant une étape d'introduction d'un miPEP dans une plante par voie exogène, ledit miPEP étant également présent naturellement dans ladite plante,
ledit miPEP introduit par voie exogène étant un peptide de 3 à 100 acides aminés, notamment de 4 à 100 acides aminés, dont la séquence comprend ou consiste en une séquence identique à celle dudit miPEP naturellement présent, laquelle séquence du miPEP naturellement présent est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante,
la somme de la quantité dudit miPEP introduit par voie exogène et de celle dudit miPEP naturellement présent étant strictement supérieure à la quantité dudit miPEP naturellement présent.

Dans un mode de réalisation, l'invention concerne un procédé tel que défini ci-dessus dans laquelle ledit gène impliqué dans la nodulation code un facteur de transcription de la famille AP2.

Dans un mode de réalisation, l'invention concerne un procédé tel que défini ci-dessus, dans lequel ledit gène impliqué dans la nodulation est le facteur de transcription *NNC1.*

Dans un mode de réalisation, l'invention concerne un procédé tel que défini ci-dessus, dans lequel ledit gène impliqué dans la nodulation est le gène *NSP1.*

Dans un mode de réalisation, l'invention concerne un procédé tel que défini ci-dessus, dans lequel ledit gène impliqué dans la nodulation est le gène *NIN.*

Dans un mode de réalisation, l'invention concerne un procédé tel que défini ci-dessus, dans lequel ledit gène impliqué dans la nodulation est le gène *ENOD40-1.*

Dans un mode de réalisation, l'invention concerne un procédé tel que défini ci-dessus, dans lequel ledit gène impliqué dans la nodulation est le gène *Hb2.*

Dans un mode de réalisation, l'invention concerne un procédé tel que défini ci-dessus, dans lequel ledit gène impliqué dans la nodulation est le gène *nifH.*

Dans un mode de réalisation, l'invention concerne un procédé tel que défini ci-dessus, dans lequel ledit miARN est le miR172c, en particulier, dans laquelle ledit miR172c possède une séquence nucléotidique comprenant ou consistant en SEQ ID NO : 1.

Dans un mode de réalisation, l'invention concerne un procédé tel que défini ci-dessus, dans lequel ledit miPEP est le miPEP172c, en particulier, dans laquelle ledit miPEP172c possède une séquence d'acides aminés comprenant ou consistant en SEQ ID NO : 2.

Dans un mode de réalisation, est décrit un procédé tel que défini ci-dessus, dans lequel ledit miARN est le miR167c, en particulier, dans laquelle ledit miR167c possède une séquence nucléotidique comprenant ou consistant en SEQ ID NO : 6.

Dans un mode de réalisation, est décrit un procédé tel que défini ci-dessus, dans lequel ledit miPEP est le miPEP167c, en particulier, dans laquelle ledit miPEP167c possède une séquence d'acides aminés comprenant ou consistant en SEQ ID NO : 7.

Dans un mode de réalisation, l'invention concerne un procédé tel que défini ci-dessus, dans lequel ladite plante est une plante légumineuse, telle que le Lotus *(Lotus sp.)* le soja *(Glycine max),* l'arachide *(Arachis hypogaea),* le haricot *(Phaseolus vulgaris),* le pois *(Pisum sativum),* la lentille *(Lens culinaris),* le pois chiche *(Cicer arietinum),* la fève et la féverolle *(Vicia faba*), les vesces *(Vicia* sp.), les gesses *(Lathyrus* sp.), la luzerne *(Medicago* sp.), le trèfle *(Trifolium* sp.), le lupin *(Lupinus* sp.), le haricot mungo (*Vigna radiata),* la réglisse *(Glycyrrhiza glabra),* le palissandre *(Dalbergia),* le lotier corniculé *(Lotus corniculatus),* le sainfoin *(Onobrychis viciifolia*), le rooibos *(Aspalathus linearis),* le fenugrec *(Trigonella foenum-graecum*).

Dans un mode de réalisation, l'invention concerne un procédé tel que défini ci-dessus, dans laquelle ladite plante est la betterave sucrière (*Beta vulgaris*).

Dans un mode de réalisation, l'invention concerne un procédé tel que défini ci-dessus, dans laquelle ladite bactérie est une bactérie de la famille des *Rhizobiaceae.*

Dans un mode de réalisation particulier, l'invention concerne un procédé tel que défini ci-dessus, dans laquelle ladite bactérie est choisie parmi les genres *Rhizobium, Sinorhizobium, Mesorhizobium, Bradyrhizobium* ou *Azorhizobium.*

Dans un mode de réalisation, l'invention concerne un procédé tel que défini ci-dessus, dans lequel ladite bactérie est une bactérie choisie parmi *Rhizobium leguminosarum, Rhizobium etli, Rhizobium tropici, Rhizobium galegae, Sinorhizobium* sp. NGR234, *Sinorhizobium meliloti, Sinorhizobium fredii, Sinorhizobium saheli, Sinorhizobium teranga, Mesorhizobium ciceri, Mesorhizobium huakuii, Mesorhizobium loti, Bradyrhizobium elkanii, Bradyrhizobium japonicum, Bradyrhizobium lupini, Bradyrhizobium* sp. "cowpea'' et *Azorhizobium caulinodans.*

Dans un mode de réalisation, l'invention concerne un procédé tel que défini ci-dessus, pour favoriser la nodulation entre une plante *Glycine max* et une bactérie *Bradirhizobium japonicum,* dans lequel le miPEP172c est introduit par voie exogène dans ladite plante *Glycine max,* ledit miPEP172c étant également naturellement présent dans ladite plante *Glycine max,*
ledit miPEP172c introduit par voie exogène étant un peptide comprenant ou consistant en une séquence identique à celle dudit miPEP172c naturellement présent, lequel miPEP172c naturellement présent est un peptide de 3 à 100 acides aminés dont la séquence est codée par un cadre ouvert de lecture situé en 5' sur le transcrit primaire du miR172c,
ledit miPEP172c étant capable d'augmenter l'accumulation dudit miR172c, lequel miR172c régule l'expression d'au moins un gène impliqué dans la nodulation chez *Glycine max,*
la somme de la quantité dudit miPEP172c introduit par voie exogène et de celle dudit miPEP172c naturellement présent étant strictement supérieure à la quantité dudit miPEP 172c naturellement présent.

Dans un mode de réalisation, est décrit un procédé tel que défini ci-dessus, pour favoriser la nodulation entre une plante *Glycine max* et une bactérie *Bradirhizobium japonicum,* dans lequel le miPEP167c est introduit par voie exogène dans ladite plante *Glycine max,* ledit miPEP167c étant également naturellement présent dans ladite plante *Glycine max,*
ledit miPEP167c introduit par voie exogène étant un peptide comprenant ou consistant en une séquence identique à celle dudit miPEP167c naturellement présent, lequel miPEP167c naturellement présent est un peptide de 3 à 100 acides aminés dont la séquence est codée par un cadre ouvert de lecture situé en 5' sur le transcrit primaire du miR167c,
ledit miPEP167c étant capable d'augmenter l'accumulation dudit miR167c, lequel miR167c régule l'expression d'au moins un gène impliqué dans la nodulation chez *Glycine max,*
la somme de la quantité dudit miPEP167c introduit par voie exogène et de celle dudit miPEP167c naturellement présent étant strictement supérieure à la quantité dudit miPEP167c naturellement présent.

Dans un mode de réalisation, l'invention concerne un procédé tel que défini ci-dessus, dans lequel ledit miPEP est introduit par voie externe dans la plante, de préférence par arrosage, par pulvérisation ou par l'ajout d'un engrais, d'un terreau, d'un substrat de culture ou à l'aide d'un support en contact avec la plante.

Dans un mode de réalisation, l'invention concerne un procédé tel que défini ci-dessus, dans lequel ledit miPEP est introduit par voie externe dans une plante, par l'ajout de granulés.

Dans un mode de réalisation, l'invention concerne un procédé tel que défini ci-dessus, dans lequel ledit miPEP est introduit par voie externe dans une graine ou une semence, de préférence par arrosage, par pulvérisation ou par l'ajout d'un engrais, d'un terreau, d'un substrat de culture ou à l'aide d'un support en contact avec la graine ou la semence.

Dans un mode de réalisation, l'invention concerne le procédé tel que défini ci-dessus, dans lequel ledit miPEP est utilisé pour traiter la plante sous forme de graine.

Dans un mode de réalisation, l'invention concerne un procédé tel que défini ci-dessus, dans lequel ledit miPEP est administré à la plante sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP.

Dans un mode de réalisation, l'invention concerne un procédé tel que défini ci-dessus, dans lequel ledit miPEP est introduit dans la plante par le biais d'un acide nucléique codant ledit miPEP, ledit acide nucléique étant introduit dans la plante.

Dans un mode de réalisation, l'invention concerne un procédé tel que défini ci-dessus, dans laquelle le nombre de nodules est augmenté chez la plante dans laquelle a été introduit ledit miPEP par rapport au nombre de nodules d'une plante identique et de même âge dans laquelle aucun miPEP n'a été introduit, ou bien par rapport au nombre de nodules d'une plante identique et de même âge dans laquelle ledit miPEP n'a pas été introduit.

Dans un mode de réalisation, l'invention concerne un procédé tel que défini ci-dessus, dans laquelle la concentration d'azote est augmentée dans les parties aériennes de la plante dans laquelle a été introduit ledit miPEP par rapport à la concentration d'azote dans les parties aériennes d'une plante identique et de même âge dans laquelle aucun miPEP n'a été introduit, ou bien par rapport à la concentration d'azote dans les parties aériennes d'une plante identique et de même âge dans laquelle ledit miPEP n'a pas été introduit.

Dans un mode de réalisation, l'invention concerne un procédé tel que défini ci-dessus, dans laquelle le poids des gousses est augmenté chez la plante dans laquelle a été introduit ledit miPEP par rapport au poids des gousses d'une plante identique et de même âge dans laquelle aucun miPEP n'a été introduit, ou bien par rapport au poids des gousses d'une plante identique et de même âge dans laquelle ledit miPEP n'a pas été introduit.

Dans un autre aspect, l'invention concerne un procédé de production d'une plante transgénique comprenant:
a) une étape d'introduction d'un acide nucléique codant un miPEP de 3 à 100 acides aminés, notamment de 4 à 100 acides aminés, dans une plante, ou dans au moins une cellule de ladite plante, dans des conditions permettant l'expression dudit miPEP,
   ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans la nodulation, et
b) une étape de culture de la plante, ou d'au moins une cellule de ladite plante, obtenues à l'étape a) dans des conditions permettant l'obtention d'une plante transgénique.

Dans un mode de réalisation, l'invention concerne un procédé de production d'une plante transgénique tel que défini ci-dessus, dans lequel ladite plante transgénique obtenue à l'étape b) est plus apte à la nodulation par rapport à une plante identique dans laquelle ledit acide nucléique n'a pas été introduit.

Dans un mode de réalisation, l'invention concerne un procédé de production d'une plante transgénique tel que défini ci-dessus, dans lequel l'expression dudit miPEP codé par l'acide nucléique introduit dans la plante entraîne une nodulation améliorée par rapport à une plante identique dans laquelle ledit acide nucléique n'a pas été introduit.

Dans un mode de réalisation, l'invention concerne un procédé de production d'une plante transgénique tel que défini ci-dessus, dans lequel l'étape a) est réalisée à l'aide d'un vecteur contenant ledit acide nucléique, de préférence un plasmide.

Dans un mode de réalisation, l'invention concerne un procédé de production d'une plante transgénique tel que défini ci-dessus, dans lequel ledit acide nucléique ne comprend pas la séquence complète dudit miR.

Dans un mode de réalisation, l'invention concerne un procédé de production d'une plante transgénique tel que défini ci-dessus, dans lequel l'expression dudit acide nucléique de l'étape a) est placée sous le contrôle d'un promoteur fort, de préférence un promoteur fort constitutif tel que le promoteur 35S.

Dans un mode de réalisation, l'invention concerne un procédé de production d'une plante transgénique tel que défini ci-dessus, dans lequel ledit gène impliqué dans la nodulation code un facteur de transcription de la famille AP2.

Dans un mode de réalisation, l'invention concerne un procédé de production d'une plante transgénique tel que défini ci-dessus, dans lequel ledit gène impliqué dans la nodulation est un gène endogène et code un facteur de transcription de la famille AP2.

Dans un mode de réalisation, l'invention concerne un procédé de production d'une plante transgénique tel que défini ci-dessus, dans lequel ledit gène impliqué dans la nodulation code le facteur de transcription NNC1.

Dans un mode de réalisation, l'invention concerne un procédé de production d'une plante transgénique tel que défini ci-dessus, dans lequel ledit gène impliqué dans la nodulation est un gène endogène et code le facteur de transcription NNC1.

Dans un mode de réalisation, l'invention concerne un procédé de production d'une plante transgénique tel que défini ci-dessus, dans lequel ledit gène impliqué dans la nodulation est le gène *NSP1.*

Dans un mode de réalisation, l'invention concerne un procédé de production d'une plante transgénique tel que défini ci-dessus, dans lequel ledit gène impliqué dans la nodulation est un gène endogène *NSP1.*

Dans un mode de réalisation, l'invention concerne un procédé de production d'une plante transgénique tel que défini ci-dessus, dans lequel ledit gène impliqué dans la nodulation est le gène *NIN.*

Dans un mode de réalisation, l'invention concerne un procédé de production d'une plante transgénique tel que défini ci-dessus, dans lequel ledit gène impliqué dans la nodulation est un gène endogène *NIN.*

Dans un mode de réalisation, l'invention concerne un procédé de production d'une plante transgénique tel que défini ci-dessus, dans lequel ledit gène impliqué dans la nodulation est le gène *ENOD40-1.*

Dans un mode de réalisation, l'invention concerne un procédé de production d'une plante transgénique tel que défini ci-dessus, dans lequel ledit gène impliqué dans la nodulation est un gène endogène *ENOD40-1.*

Dans un mode de réalisation, l'invention concerne un procédé de production d'une plante transgénique tel que défini ci-dessus, dans lequel ledit gène impliqué dans la nodulation est le gène *Hb2.*

Dans un mode de réalisation, l'invention concerne un procédé de production d'une plante transgénique tel que défini ci-dessus, dans lequel ledit gène impliqué dans la nodulation est le gène endogène *Hb2.*

Dans un mode de réalisation, l'invention concerne un procédé de production d'une plante transgénique tel que défini ci-dessus, dans lequel ledit gène impliqué dans la nodulation est le gène *nifH.*

Dans un mode de réalisation, l'invention concerne un procédé de production d'une plante transgénique tel que défini ci-dessus, dans lequel ledit gène impliqué dans la nodulation est le gène endogène *nifH.*

Dans un mode de réalisation, l'invention concerne un procédé de production d'une plante transgénique tel que défini ci-dessus, dans lequel ledit miARN est le miR172c, en particulier, dans lequel ledit miR172c possède une séquence nucléotidique comprenant ou consistant en SEQ ID NO : 1.

Dans un mode de réalisation, l'invention concerne un procédé de production d'une plante transgénique tel que défini ci-dessus, dans lequel ledit miPEP est le miPEP172c, en particulier, dans lequel ledit miPEP172c possède une séquence d'acides aminés comprenant ou consistant en SEQ ID NO : 2.

Dans un mode de réalisation, l'invention concerne un procédé de production d'une plante transgénique tel que défini ci-dessus, dans lequel ledit acide nucléique introduit à l'étape a) comprend une séquence nucléotidique consistant en SEQ ID NO : 3.

Dans un mode de réalisation, est décrit un procédé de production d'une plante transgénique tel que défini ci-dessus, dans lequel ledit miARN est le miR167c, en particulier, dans lequel ledit miR167c possède une séquence nucléotidique comprenant ou consistant en SEQ ID NO : 6.

Dans un mode de réalisation, est décrit un procédé de production d'une plante transgénique tel que défini ci-dessus, dans lequel ledit miPEP est le miPEP167c, en particulier, dans lequel ledit miPEP167c possède une séquence d'acides aminés comprenant ou consistant en SEQ ID NO : 7.

Dans un mode de réalisation, est décrit un procédé de production d'une plante transgénique tel que défini ci-dessus, dans lequel ledit acide nucléique introduit à l'étape a) comprend une séquence nucléotidique consistant en SEQ ID NO : 8.

Dans un mode de réalisation, l'invention concerne un procédé tel que défini ci-dessus, dans lequel ladite plante est une plante légumineuse, telle que le Lotus *(Lotus sp.)* le soja (*Glycine max*), l'arachide *(Arachis hypogaea),* le haricot *(Phaseolus vulgaris),* le pois *(Pisum sativum),* la lentille (*Lens culinaris),* le pois chiche *(Cicer arietinum),* la fève et la féverolle *(Vicia faba*), les vesces *(Vicia* sp.), les gesses *(Lathyrus* sp.), la luzerne *(Medicago* sp.), le trèfle *(Trifolium* sp.), le lupin *(Lupinus* sp.), le haricot mungo (*Vigna radiata),* la réglisse *(Glycyrrhiza glabra),* le palissandre *(Dalbergia),* le lotier corniculé *(Lotus corniculatus),* le sainfoin *(Onobrychis viciifolia*), le rooibos *(Aspalathus linearis),* le fenugrec *(Trigonella foenum-graecum*).

Dans un mode de réalisation, l'invention concerne un procédé tel que défini ci-dessus, dans laquelle ladite plante est la betterave sucrière (*Beta vulgaris*).

Dans un mode de réalisation, l'invention concerne un procédé tel que défini ci-dessus, dans laquelle ladite bactérie est une bactérie de la famille des *Rhizobiaceae.*

Dans un mode de réalisation particulier, l'invention concerne un procédé tel que défini ci-dessus, dans laquelle ladite bactérie est choisie parmi les genres *Rhizobium, Sinorhizobium, Mesorhizobium, Bradyrhizobium* ou *Azorhizobium.*

Dans un mode de réalisation, l'invention concerne un procédé tel que défini ci-dessus, dans lequel ladite bactérie est une bactérie choisie parmi *Rhizobium leguminosarum, Rhizobium etli, Rhizobium tropici, Rhizobium galegae, Sinorhizobium* sp. NGR234, *Sinorhizobium meliloti, Sinorhizobium fredii, Sinorhizobium saheli, Sinorhizobium teranga, Mesorhizobium ciceri, Mesorhizobium huakuii, Mesorhizobium loti, Bradyrhizobium elkanii, Bradyrhizobium japonicum, Bradyrhizobium lupini, Bradyrhizobium* sp. "cowpea'' et *Azorhizobium caulinodans.*

Dans un mode de réalisation, l'invention concerne un procédé de production d'une plante transgénique tel que défini ci-dessus, comprenant :
**a)** une étape d'introduction d'un acide nucléique contenant la séquence nucléotidique SEQ ID NO : 3, codant le miPEP172c consistant en la séquence d'acides aminés SEQ ID NO : 2, dans une plante *Glycine max,* ou dans au moins une cellule de ladite plante *Glycine max,* dans des conditions permettant l'expression du miPEP172c,
   ledit miPEP172c étant également naturellement présent dans ladite plante *Glycine max,* ledit miPEP naturellement présent étant un peptide dont la séquence est codée par un cadre ouvert de lecture situé en 5' sur le transcrit primaire du miR172c, ledit miPEP172c étant capable de moduler l'accumulation dudit miR172c, lequel miR172c régule l'expression d'au moins un gène impliqué dans la nodulation chez *Glycine max,* et
**b)** une étape de culture de la plante, ou d'au moins une cellule de ladite plante, obtenues à l'étape a) dans des conditions permettant l'obtention d'une plante *Glycine max* transgénique.

Dans un mode de réalisation, est décrit un procédé de production d'une plante transgénique tel que défini ci-dessus, comprenant :
**a)** une étape d'introduction d'un acide nucléique contenant la séquence nucléotidique SEQ ID NO : 8, codant le miPEP167c consistant en la séquence d'acides aminés SEQ ID NO : 7, dans une plante *Glycine max,* ou dans au moins une cellule de ladite plante *Glycine max,* dans des conditions permettant l'expression du miPEP167c,
   ledit miPEP167c étant également naturellement présent dans ladite plante *Glycine max,* ledit miPEP naturellement présent étant un peptide dont la séquence est codée par un cadre ouvert de lecture situé en 5' sur le transcrit primaire du miR167c, ledit miPEP167c étant capable de moduler l'accumulation dudit miR167c, lequel miR167c régule l'expression d'au moins un gène impliqué dans la nodulation chez *Glycine max,* et
**b)** une étape de culture de la plante, ou d'au moins une cellule de ladite plante, obtenues à l'étape a) dans des conditions permettant l'obtention d'une plante *Glycine max* transgénique.

Dans un mode de réalisation, l'invention concerne un procédé de production tel que défini ci-dessus, dans lequel ledit miPEP est introduit dans la plante par le biais d'un acide nucléique codant ledit miPEP, ledit acide nucléique étant introduit dans la plante.

Dans un mode de réalisation, l'invention concerne un procédé de production d'une plante transgénique tel que défini ci-dessus, dans laquelle le nombre de nodules est augmenté chez la plante dans laquelle a été introduit ledit miPEP par rapport au nombre de nodules chez une plante identique et de même âge dans laquelle aucun miPEP n'a été introduit, ou bien par rapport au nombre de nodules d'une plante identique et de même âge dans laquelle ledit miPEP n'a pas été introduit.

Dans un mode de réalisation, l'invention concerne un procédé de production d'une plante transgénique tel que défini ci-dessus, dans laquelle la concentration d'azote est augmentée dans les parties aériennes de la plante dans laquelle a été introduit ledit miPEP par rapport à la concentration d'azote dans les parties aériennes d'une plante identique et de même âge dans laquelle aucun miPEP n'a été introduit, ou bien par rapport à la concentration d'azote dans les parties aériennes d'une plante identique et de même âge dans laquelle ledit miPEP n'a pas été introduit.

Dans un mode de réalisation, l'invention concerne un procédé de production d'une plante transgénique tel que défini ci-dessus, dans laquelle le poids des gousses est augmenté chez la plante dans laquelle a été introduit ledit miPEP par rapport au poids des gousses d'une plante identique et de même âge dans laquelle aucun miPEP n'a été introduit, ou bien par rapport au poids des gousses d'une plante identique et de même âge dans laquelle ledit miPEP n'a pas été introduit.

Dans un aspect, l'invention concerne également une plante transgénique telle qu'obtenue par le procédé de production tel que défini ci-dessus.

Dans un autre aspect, l'invention concerne une plante dans laquelle a été introduit un miPEP selon l'utilisation ou le procédé pour favoriser la nodulation décrits ci-dessus.

Dans un autre aspect, l'invention concerne un peptide ayant une séquence d'acides aminés présentant au moins 80% d'identité, de préférence au moins 90 % d'identité, avec la séquence d'acides aminés SEQ ID NO : 2.

Dans un mode de réalisation particulier, l'invention concerne un peptide comprenant ou consistant de séquence SEQ ID NO : 2.

Dans un mode de réalisation particulier, ledit peptide de séquence SEQ ID NO : 2 est un peptide isolé et/ou purifié, un peptide synthétique ou un peptide recombinant.

Dans un autre aspect, l'invention concerne une composition, en particulier une composition phytosanitaire, comprenant le miPEP172c en tant que substance active, ledit miPEP172c consistant de préférence en SEQ ID NO : 2.

Dans un autre aspect, l'invention concerne un peptide ayant une séquence d'acides aminés présentant au moins 80% d'identité, de préférence au moins 90 % d'identité, avec la séquence d'acides aminés SEQ ID NO : 7.

Dans un mode de réalisation particulier, l'invention concerne un peptide comprenant ou consistant de séquence SEQ ID NO : 7.

Dans un mode de réalisation particulier, ledit peptide de séquence SEQ ID NO : 7 est un peptide isolé et/ou purifié, un peptide synthétique ou un peptide recombinant.

Dans un mode de réalisation, l'invention concerne un peptide tel que défini ci-dessus, ledit peptide étant marqué.

De manière non limitative, un peptide marqué peut notamment être obtenu en fusionnant ledit peptide à une autre séquence peptidique, à un marqueur fluorescent (GUS, GFP, LacZ ...), à une séquence signal (permettant l'adressage du peptide), à une séquence Tag (permettant la purification du miPEP) ou encore à un isotope radioactif.

Dans le cas où le peptide de l'invention est marqué à l'aide d'une autre séquence peptidique, celle-ci n'est pas prise en compte pour calculer un pourcentage d'identité par rapport au peptide de l'invention.

Dans un autre aspect, est décrite une composition, en particulier une composition phytosanitaire, comprenant le miPEP167c en tant que substance active, ledit miPEP167c consistant de préférence en SEQ ID NO : 7.

Dans un autre aspect, l'invention concerne une composition telle que définie ci-dessus, dans laquelle ledit miPEP172c est à une concentration de 10⁻⁹ M à 10⁻⁴ M, en particulier 10⁻⁹, 10⁻⁸, 10⁻⁷, 10⁻⁶, 10⁻⁵ ou 10⁻⁴ M.

De préférence, une composition telle que définie ci-dessus a une concentration de 10⁻⁸ M à 10⁻⁵ M pour une application par arrosage ou par pulvérisation sur la plante ou par l'ajout d'un engrais, d'un terreau, d'un substrat de culture ou à l'aide d'un support en contact avec la plante.

De préférence, une composition telle que définie ci-dessus a une concentration de 10⁻⁸ M à 10⁻⁵ M pour une application par arrosage ou par pulvérisation sur la graine ou par l'ajout d'un engrais, d'un terreau, d'un substrat de culture ou à l'aide d'un support en contact avec la graine ou la semence.

De manière complémentaire, des compositions plus ou moins concentrées peuvent être envisagées pour traiter la plante avec le miPEP. Par exemple, et de manière non limitative, des compositions plus concentrées comprenant 10⁻¹ M à 10⁻³ M, notamment 10⁻² M de miPEP, peuvent être utilisées dans le cas où le miPEP introduit par voie exogène est administré à la plante par épandage.

Dans un autre aspect, l'invention concerne une composition telle que définie ci-dessus, comprenant de plus un excipient, un diluant ou un solvant.

Dans un mode de réalisation, l'invention concerne une composition telle que définie ci-dessus formulée de façon à former un enrobé.

Dans un mode de réalisation, l'invention concerne une composition telle que définie ci-dessus :
- sous la forme d'un granulé,
- formulée de façon à former un granulé, ou
- formulée de façon à être contenue dans un granulé.

Dans un autre aspect, l'invention concerne une composition comprenant en combinaison une quantité de semences d'une plante et une quantité d'un peptide dont la séquence comprend ou consiste en une séquence identique à celle d'un miPEP naturellement présent chez ladite plante.

Dans un mode de réalisation, l'invention concerne une composition comprenant en combinaison une quantité de semences d'une plante, notamment *Glycine max,* et une quantité d'un peptide dont la séquence comprend ou consiste en une séquence identique à celle du miPEP172c.

Dans un mode de réalisation, est décrite une composition comprenant en combinaison une quantité de semences d'une plante, notamment *Glycine max,* et une quantité d'un peptide dont la séquence comprend ou consiste en une séquence identique à celle du miPEP167c. Dans un autre aspect, l'invention concerne une composition telle que définie ci-dessus, comprenant de plus un excipient, un diluant ou un solvant.

Dans un mode de réalisation, l'invention concerne une composition telle que définie ci-dessus formulée de façon à former une semence enrobée ou une graine enrobée.

L'enrobage peut être réalisé selon les procédés classiquement utilisés dans l'industrie agroalimentaire et peut être obtenu en utilisant un matériau apte à se désagréger dans un solvant ou dans la terre, tel qu'un liant ou de l'argile.

La composition contenant ledit miPEP peut notamment être appliquée sur la semence, sous, dans ou sur la couche d'enrobage.

Selon l'invention, l'enrobage peut être utilisé pour par exemple conférer des propriétés particulières à une composition de miPEP, ou encore à une composition de semences en combinaison avec un miPEP.

Dans un autre aspect, l'invention concerne une semence enrobée avec une composition comprenant un miPEP capable de moduler l'accumulation d'un miR chez la plante issue de la semence, lequel miR régule l'expression d'au moins un gène impliqué dans la nodulation chez ladite plante.

Dans un mode de réalisation, l'invention concerne une semence enrobée avec une composition comprenant le miPEP172c.

Dans un mode de réalisation, l'invention concerne une semence enrobée avec une composition comprenant un peptide dont la séquence présente au moins 80% d'identité, de préférence au moins 90% d'identité avec la séquence SEQ ID NO : 2.

Dans un mode de réalisation, l'invention concerne une semence enrobée avec une composition comprenant le peptide SEQ ID NO : 2.

Dans un mode de réalisation, est décrite une semence enrobée avec une composition comprenant le miPEP167c.

Dans un mode de réalisation, est décrite une semence enrobée avec une composition comprenant un peptide dont la séquence présente au moins 80% d'identité, de préférence au moins 90% d'identité avec la séquence SEQ ID NO : 7.

Dans un mode de réalisation, est décrite une semence enrobée avec une composition comprenant le peptide SEQ ID NO : 7.

Dans un mode de réalisation, l'invention concerne une semence enrobée telle que définie ci-dessus, ladite plante étant une plante légumineuse, telle que le Lotus *(Lotus sp.)* le soja *(Glycine max),* l'arachide *(Arachis hypogaea),* le haricot *(Phaseolus vulgaris),* le pois *(Pisum sativum),* la lentille (*Lens culinaris*), le pois chiche *(Cicer arietinum),* la fève et la féverolle *(Vicia faba*), les vesces *(Vicia* sp.), les gesses *(Lathyrus* sp.), la luzerne *(Medicago* sp.), le trèfle *(Trifolium* sp.), le lupin *(Lupinus* sp.), le haricot mungo (*Vigna radiata),* la réglisse *(Glycyrrhiza glabra),* le palissandre *(Dalbergia),* le lotier corniculé *(Lotus corniculatus),* le sainfoin *(Onobrychis viciifolia*), le rooibos *(Aspalathus linearis),* le fenugrec *(Trigonella foenum-graecum*).

Dans un autre aspect, l'invention concerne un protocole de production d'un peptide recombinant, dont la séquence comprend ou consiste en une séquence identique à celle d'un miPEP tel que défini ci-dessus, comprenant une étape de transformation d'un organisme avec un vecteur d'expression codant ledit peptide recombinant.

Dans un mode de réalisation, ledit organisme est choisi parmi le groupe comprenant les bactéries, les levures, les champignons (autre que levures), les cellules animales, les plantes et les animaux.

Dans un mode de réalisation, ledit organisme est *Escherichia coli.*

En particulier, l'invention concerne un protocole de production d'un peptide recombinant tel que défini ci-dessus, comprenant les étapes suivantes :
- l'acide nucléique codant ledit peptide recombinant est lié à un acide nucléique codant une étiquette, telle que la GST,
- le vecteur d'expression contenant ledit acide nucléique codant ledit peptide recombinant est introduit dans la bactérie *E. coli,*
- la bactérie *E. coli* contenant le vecteur d'expression est cultivée dans du milieu LB de préférence jusqu'à une DO comprise entre 0.2 et 0.4,
- la production du peptide recombinant est induite avec de l'IPTG, de préférence pendant 4 à 5 heures,
- les bactéries *E. coli* sont centrifugées et lysées,
- le surnageant est filtré,
- ledit peptide recombinant est purifié sur une colonne d'affinité glutathion sepharose,
- si nécessaire, cliver la GST avec une protéase.

Dans un autre aspect, l'invention concerne une bactérie transformée avec une séquence codant un miPEP tel que défini précédemment.

Dans un mode de réalisation, l'invention concerne une bactérie transformée avec une séquence codant un miPEP, ladite bactérie étant une bactérie de la famille des *Rhizobiaceae.* Dans un mode de réalisation, l'invention concerne une bactérie transformée avec une séquence codant un miPEP, ladite bactérie étant choisie parmi les genres *Rhizobium, Sinorhizobium, Mesorhizobium, Bradyrhizobium* ou *Azorhizobium.*

Dans un mode de réalisation, l'invention concerne une bactérie transformée avec une séquence codant un miPEP, ladite bactérie étant choisie parmi *Rhizobium leguminosarum, Rhizobium etli, Rhizobium tropici, Rhizobium galegae, Sinorhizobium* sp. NGR234, *Sinorhizobium meliloti, Sinorhizobium fredii, Sinorhizobium saheli, Sinorhizobium teranga, Mesorhizobium ciceri, Mesorhizobium huakuii, Mesorhizobium loti, Bradyrhizobium elkanii, Bradyrhizobium japonicum, Bradyrhizobium lupini, Bradyrhizobium* sp. "cowpea'' et *Azorhizobium caulinodans.*

Dans un autre aspect, l'invention concerne un anticorps reconnaissant spécifiquement le miPEP172c, en particulier ledit miPEP172c consistant en SEQ ID NO : 2.

Un tel anticorps peut être obtenu à partir d'un procédé connu de l'homme du métier, comme par exemple en injectant ledit miPEP172c à un animal non humain pour déclencher une réaction d'immunisation et la production d'anticorps par ledit animal.

Dans un autre aspect, l'invention concerne un procédé d'immunolocalisation du miPEP172c comprenant une étape de marquage d'un échantillon biologique d'une plante avec un anticorps reconnaissant spécifiquement ledit miPEP172c.

Dans un autre aspect, est décrit un anticorps reconnaissant spécifiquement le miPEP167c, en particulier ledit miPEP167c consistant en SEQ ID NO : 7.

Un tel anticorps peut être obtenu à partir d'un procédé connu de l'homme du métier, comme par exemple en injectant ledit miPEP167c à un animal non humain pour déclencher une réaction d'immunisation et la production d'anticorps par ledit animal.

Dans un autre aspect, est décrit un procédé d'immunolocalisation du miPEP167c comprenant une étape de marquage d'un échantillon biologique d'une plante avec un anticorps reconnaissant spécifiquement ledit miPEP167c.

Dans un autre aspect, l'invention concerne un procédé de culture de bactéries, comprenant une étape de mise en contact desdites bactéries:
- avec un mélange comprenant une plante ou une partie de plante, en particulier une culture de racine, et un peptide dont la séquence comprend ou consiste en une séquence identique à celle d'un miPEP naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante, ou
- avec une plante transgénique telle que définie précédemment,
la plante, la partie de plante, et la plante transgénique étant aptes à former une symbiose avec ladite bactérie.

Dans un mode de réalisation particulier, l'invention concerne le procédé tel que défini ci-dessus dans lequel les bactéries cultivées appartiennent à la famille des *Rhizobiaceae.*

Dans un mode de réalisation particulier, l'invention concerne le procédé tel que défini ci-dessus dans lequel lesdites bactéries sont choisies parmi les genres *Rhizobium, Sinorhizobium, Mesorhizobium, Bradyrhizobium* ou *Azorhizobium.*

Dans un mode de réalisation particulier, l'invention concerne le procédé tel que défini ci-dessus dans lequel lesdites bactéries sont choisies parmi *Rhizobium leguminosarum, Rhizobium etli, Rhizobium tropici, Rhizobium galegae, Sinorhizobium* sp. NGR234, *Sinorhizobium meliloti, Sinorhizobium fredii, Sinorhizobium saheli, Sinorhizobium teranga, Mesorhizobium ciceri, Mesorhizobium huakuii, Mesorhizobium loti, Bradyrhizobium elkanii, Bradyrhizobium japonicum, Bradyrhizobium lupini, Bradyrhizobium* sp. "cowpea'' et *Azorhizobium caulinodans.*

En particulier, le procédé de culture desdites bactéries tel que défini ci-dessus est réalisé dans des conditions de culture permettant la croissance, voire l'amélioration de la croissance de la plante, de la partie de plante, et de la plante transgénique, et celle des bactéries.

En particulier, le procédé de culture des bactéries tel que défini ci-dessus est réalisé dans des conditions de culture permettant la symbiose entre la bactérie et la plante, la partie de plante ou la plante transgénique.

Dans un mode de réalisation, le peptide présent dans le mélange est un peptide isolé, un peptide isolé et/ou purifié, un peptide synthétique ou un peptide recombinant.

Dans un mode de réalisation, l'invention concerne un procédé de culture de bactéries tel que défini ci-dessus, dans lequel ledit miR est le miR172c, en particulier, dans laquelle ledit miR172c possède une séquence nucléotidique consistant en SEQ ID NO : 1.

Dans un mode de réalisation, l'invention concerne un procédé de culture de bactéries tel que défini ci-dessus, dans lequel ledit miPEP est le miPEP172c, en particulier, dans laquelle ledit miPEP172c possède une séquence d'acides aminés consistant en SEQ ID NO : 2.

Dans un mode de réalisation, est décrit un procédé de culture de bactéries tel que défini ci-dessus, dans lequel ledit miR est le miR167c, en particulier, dans laquelle ledit miR167c possède une séquence nucléotidique consistant en SEQ ID NO : 6.

Dans un mode de réalisation, est décrit un procédé de culture de bactéries tel que défini ci-dessus, dans lequel ledit miPEP est le miPEP167c, en particulier, dans laquelle ledit miPEP167c possède une séquence d'acides aminés consistant en SEQ ID NO : 7.

En particulier, l'invention concerne un procédé de culture de bactéries tel que défini ci-dessus, dans lequel ladite partie de plante est une racine ou un fragment de racines.

Dans un autre aspect, l'invention concerne un procédé pour produire de l'inoculum de bactéries, en particulier des bactéries de la famille des *Rhizobiaceae,* comprenant :
- une étape de co-culture de bactéries avec une matière végétale vivante, dite plante hôte, correspondant au moins en partie, à une partie constitutive de racine d'une plante apte à former une symbiose avec lesdites bactéries, et
- une étape de mise en contact d'une quantité d'un peptide avec la susdite co-culture,
ledit peptide ayant une séquence comprenant ou consistant en une séquence identique à celle d'un miPEP naturellement présent dans ladite plante hôte, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante hôte.

L'invention concerne également un procédé pour produire de l'inoculum de bactéries, en particulier des bactéries de la famille des *Rhizobiaceae,* comprenant une étape de co-culture des bactéries avec une matière végétale vivante, dite plante hôte, correspondant au moins en partie, à une partie constitutive de racine d'une plante apte à former une symbiose avec lesdites bactéries, et
ladite plante hôte étant une plante transgénique ou une plante dans laquelle a été introduit un peptide,
ledit peptide ayant une séquence comprenant ou consistant en une séquence identique à celle d'un miPEP naturellement présent dans ladite plante hôte, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante hôte.

L'invention concerne également un procédé pour produire de l'inoculum de bactéries, en particulier des bactéries de la famille des *Rhizobiaceae,* comprenant une étape de mélange rassemblant :
- des bactéries,
- une matière végétale vivante, dite plante hôte, correspondant au moins en partie, à une partie constitutive de racine d'une plante apte à former une symbiose avec lesdites bactéries, et
- un peptide,
ledit peptide ayant une séquence comprenant ou consistant en une séquence identique à celle d'un miPEP naturellement présent dans ladite plante hôte, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante hôte.

Dans un autre aspect, l'invention concerne un inoculum de bactéries, en particulier un inoculum de bactéries de la famille des *Rhizobiaceae,* adapté à l'inoculation d'une plante hôte, comprenant au moins une bactérie et un peptide dont la séquence comprend ou consiste en une séquence identique à celle d'un miPEP naturellement présent dans la plante hôte,
ledit miPEP naturellement présent dans la plante hôte étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante hôte.

De préférence, ledit miPEP est le miPEP172c.

De préférence, ledit miPEP est le miPEP167c.

Le peptide utilisé pour produire de l'inoculum, ou le peptide présent dans l'inoculum, est notamment un peptide isolé, un peptide isolé et/ou purifié, un peptide synthétique ou un peptide recombinant.

De préférence, l'inoculum contient en plus une plante ou une partie de plante, en particulier une racine, une culture de racine ou une partie de racine.

Les séquences du miPEP172c, de son cadre ouvert de lecture, du miR172c et du transcrit primaire du miR172c chez *Glycine max* sont indiquées dans le tableau 1.

**Tableau 1.**

| | | |
|---|---|---|
| **miR172c** | GGAAUCUUGAUGAUGCUGCAG | **SEQ ID NO : 1** |
| **miPEP172c** | MWVLCLFCWPTYTHGS | **SEQ ID NO : 2** |
| **miORF172c** | ATGTGGGTCTTGTGTTTGTTTTGTTGGCCTA CATATACACATGGGAGTTGA | **SEQ ID NO : 3** |
| **pri-miR172c** | CACTCTCTCATCTCTACTTGACCACTCTCTC TATATATACACCACTAAACCCTTTCTTCGTT CTCAGTTATCTTCTTTCACTTCACTTAACCT AGCCTCCTTCCCATTCTGATTTGAGTTCTTG CTTTGCCTCTTAATTTTATTTTTCCTCTTAGC TTTCTTCGATCTTAACCTTTGAACAGTAATT GCTTGCTTTGTATACATTAATTACTATAGGT | **SEQ ID NO : 4** |
| | GGTACTTTGGCAGCTTTTCTATTGTATAGAG ACTTATCAGTGAATAATACTACTATGTGGGT CTTGTGTTTGTTTTGTTGGCCTACATATACA CATGGGAGTTGAGGAGCCTTTTAGGCCTTA GCCTTACATTAGTTGTCCCTCTTTCCTTGCCT TTACTTTTGCTTCATATATTGGATTTGATTA GATTTAGGGTTAGGGTTAGGGCTGTTCTATT CTCCTCCTCCTCCTTTTCTACTTCCTGATATG CTTCAATTCCTCCTCTCTCTCTCTCTCTCTTG AAGTTTTATGTTTTATGGCCAAGTGATCTTA ATGGTGGTAACACAGAGAGACCTAATGAAG TCCTAAATAAACTAAAGAAATCAGTCACTG TTTGCCGGTGGAGCATCATCAAGATTCACA AGCTTTAGGGGCATTAATTTGTTTGAGGTGG TTCCTTATTGCTCCAAAACCAATTAGCCCTT TTGCTATGGGAATCTTGATGATGCTGCAGC AGCAATAAATGACTAATACTACTACCAGAT ATTGCTTGAGGAGGATCTCACTACTACACA ACTGTGCTACAAGGCCAGGAGCAAAGGAG GTTATTTAATTAACTAAATAATTATGTTATT AATTTGTTTTCTAAATATTCAAGAAATTGGA TTAGGTAATTACCAAGTTACAAGCCACTTTT GGATTTACCTTTATTAACTCTAGTAGCTGAT AAAT | |

Les séquences du miPEP167c, de son cadre ouvert de lecture, du miR167c et du transcrit primaire du miR167c chez *Glycine max* sont indiquées dans le tableau 2.

**Tableau 2.**

| | | |
|---|---|---|
| **miR167c** | UGAAGCUGCCAGCAUGAUCUG | **SEQ ID NO : 6** |
| **miPEP167c** | MKGVHHFFHHKYVGLRG | **SEQ ID NO : 7** |
| **miORF167c** | ATGAAAGGGGTTCATCACTTTTTTCATCACA AGTATGTTGGTTTGAGAGGT | **SEQ ID NO : 8** |
| **pri-miR167c** | GAAAAGGAAGTCATTGTCAATAGCTGTAAG GAGGAGCAAAACCTACCATTAAATACTCCC TCTATGGCACAGTAGTGAAAGAAAAGAGAC ATTGAGCTGCGCACAGGCATTCATTCATAT GGATCAGTGTGGAGATAAAGAGGATCACGA GGGTCTCCCTTTAATTACTCCAAACATGCAT GGCTTGTTCCCCTTTTTGTTAATTTCTATGTA TCAAGATGTTGGTACCCTCTCTCAGGATTTG CTTCAATGAAAGGGGTTCATCACTTTTTTCA TCACAAGTATGTTGGTTTGAGAGGTTGAAG CTGCCAGCATGATCTGGTAAATCACATACTT TTTTTTTTCTCACCTCTCATGCCTAATTTTTA | **SEQ ID NO : 9** |
| | AGCACCAGTCATTAGAGAAAATAATGGTGA AAAATCCATCTATTCAATTTTTTTTTTCAAA TTCAAGGTTTCCAGTATGTATCACTAATGGT GAAAAAAGTGATGGAATTTTGTAGAACATG GGTTAAATTTACTTTTTTTTTTTTTGAGTTTT CATTTTCTTCAAGTTTCTGAGCCAAGAAATA AAAGAGACTTATAAATTGGAATTAATACTT AAAGGAAACCCACCAGAAGGGCAATTTGGT TATCATAAGATGTGGTTTCCATCAGGTCATC TTGCAGCTTCAATCACTCAAT | |

Les pages 36 à 50 correspondent à des extraits de la demande de brevet français n° FR 13/60727 déposée le 31 octobre 2013 pour « *Micropeptides et leur utilisation pour moduler l'expression de gènes* »

*L'objet de la demande* FR 13 60727 *concerne des micropeptides (peptides codés par des microARNs ou « miPEPs ») et leur utilisation pour moduler l'expression de gènes.*

*Les microARNs (miRs) sont des petits ARNs non codants, d'environ 21 nucléotides après maturation, qui contrôlent l'expression de gènes cibles au niveau post-transcriptionnel, en dégradant l'ARNm cible ou en inhibant sa traduction. Les miRs sont rencontrés chez les plantes et les animaux.*

*Les gènes cibles sont souvent des gènes clés de processus développementaux. Ils codent par exemple des facteurs de transcription ou des protéines du protéasome.*

*La régulation de l'expression des miRs est très peu connue, mais on sait notamment que celle-ci fait intervenir, à l'instar de la plupart des gènes codants, une ARN polymerase II : cette enzyme produit un transcrit primaire, appelé « pri-miR », qui est ensuite maturé par un complexe protéique contenant notamment les enzymes type Dicer. Cette maturation conduit tout d'abord à la formation d'un précurseur de miR appelé « pre-miR », ayant une structure secondaire en forme tige-boucle contenant le miR et sa séquence miR* complémentaire. Puis le précurseur est maturé, ce qui conduit à la formation d'un ARN double brin plus court contenant le miR et le miR*. Le miR est ensuite pris en charge par le complexe RISC qui clive l'ARNm du gène cible ou bien inhibe sa traduction.*

*Par ailleurs, il a été montré que la présence d'introns dans le transcrit primaire du microARN augmente l'expression du microARN mature (*Schwab et al., EMBO Rep., 14(7):615-21, 2013*). Cependant, du fait de difficultés expérimentales, les transcrits primaires de microARNs, ou pri-miRs, sont très peu étudiés.*

*Environ 50% des gènes eucaryotes possèdent, au sein de leur région 5'UTR (5' UnTranslated Region) en amont de la séquence codante, de petits cadres ouverts de lecture. Ces petits cadres ouverts de lecture (ou « uORFs » pour upstream ORFs) peuvent jouer un rôle de régulateur de la traduction, principalement en cis, en modulant la fixation et la vitesse des ribosomes sur l'ARNm, mais également en trans selon un mécanisme encore inconnu, par l'intermédiaire de peptides codés par lesdits uORFs (*Combier et al., Gene Dev, 22:1549-1559, 2008*). Par définition, les uORFS sont présents en amont de gènes codants.*

*Récemment, il a également été découvert de petits ORFs dans de longs ARN non codants intergéniques (lincRNAs) dont la fonction putative, si elle existe, n'est pas connue (*Ingolia et al., Cell, 147(4):789-802, 2011 *;* Guttman & Rinn, Nature, 482(7385):339-46, 2012*).*

*Toutefois, aucun exemple n'a encore été rapporté concernant l'existence d'ORFs codant des peptides au sein de microARNs non codants. Jusqu'à présent, les microARNs, et par extension leur transcrit primaire, ont toujours été considérés, de par leur mode d'action particulier, comme des ARNs régulateurs non codants ne produisant aucun peptide.*

*L'un des aspects de l'objet de la demande* FR 13 60727 *est de proposer des peptides capables de moduler l'expression des microARNs.*

*Un autre aspect de l'objet de la demande* FR 13 60727 *est de proposer un moyen permettant de moduler l'expression d'un ou plusieurs gènes cibles d'un microARN.*

*L'objet de la demande* FR 13 60727 *présente l'avantage de permettre un contrôle plus facile et plus efficace de l'expression de gènes ciblés par les microARNs, à l'aide d'un moyen autre que le microARN.*

*L'objet de la demande* FR 13 60727 *concerne ainsi un procédé de détection et d'identification d'un micropeptide (miPEP) codé par une séquence nucléotidique contenue dans la séquence du transcrit primaire d'un microARN,*
*comprenant :*
- *a) une étape de détection d'un cadre ouvert de lecture de 15 à 303 nucléotides contenu dans la séquence du transcrit primaire dudit microARN, puis*
- *b) une étape de comparaison entre :*
   - *l'accumulation dudit microARN dans une cellule eucaryote déterminée exprimant ledit microARN,*
      *en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et*
   - *l'accumulation dudit microARN dans une cellule eucaryote de même type que la susdite cellule eucaryote déterminée exprimant ledit microARN,*
   *en absence dudit peptide,*
*dans lequel, une modulation de l'accumulation dudit microARN en présence dudit peptide par rapport à l'accumulation dudit microARN en absence dudit peptide indique l'existence d'un micropeptide codé par ledit cadre ouvert de lecture.*

*Dans une première étape, le procédé de détection et d'identification d'un micropeptide consiste donc à détecter sur le transcrit primaire d'un microARN l'existence d'un cadre ouvert de lecture codant potentiellement un peptide.*

*La deuxième étape permet, quant à elle, de caractériser ledit peptide, c'est-à-dire de déterminer si ledit peptide correspond a un peptide réellement produit dans la cellule, en recherchant un effet dudit peptide sur l'accumulation dudit microARN.*

*Pour mettre en évidence un effet du peptide sur l'accumulation du microARN, une quantité importante de peptide est introduite dans une première cellule exprimant ledit microARN. L'accumulation du microARN dans cette première cellule est ensuite mesurée et comparée avec l'accumulation du microARN dans une deuxième cellule identique à la première, mais ne contenant pas ledit peptide.*

*L'observation d'une variation des quantités de microARN entre les cellules en présence et en absence du peptide indique ainsi (i) qu'il existe un peptide codé sur le transcrit primaire dudit microARN, (ii) que la séquence de ce peptide est codée par le cadre ouvert de lecture identifié sur le transcrit primaire dudit microARN, et (iii) que ledit peptide agit sur l'accumulation dudit microARN.*

*L'objet de la demande* FR 13 60727 *repose donc sur la double observation inattendue faite par les Inventeurs que d'une part, il existe des cadres ouverts de lecture susceptibles de coder des micropeptides présents sur les transcrits primaires de microARNs, et d'autre part que lesdits micropeptides sont capables de moduler l'accumulation desdits microARNs.*

*Dans la demande* FR 13 60727*, les termes « microARN », « microARN non codant » et « miR » sont équivalents et peuvent être utilisés l'un pour l'autre. Ils définissent des petites molécules d'ARN d'environ 21 nucléotides, qui ne sont pas traduites et ne conduisent pas à un peptide ou une protéine.*

*Cependant, sous cette forme mature, les microARNs assurent une fonction de régulation de certains gènes via des mécanismes post-transcriptionnels, comme par exemple par l'intermédiaire du complexe RISC.*

*Le transcrit primaire du microARN ou « pri-miR » correspond lui à la molécule d'ARN directement obtenue à partir de la transcription de la molécule d'ADN. Généralement, ce transcrit primaire subit une ou plusieurs modifications post-transcriptionnelles, qui entraînent par exemple une structure particulière de l'ARN ou un clivage de certaines parties de l'ARN par des phénomènes d'épissage, et qui conduisent à la forme précurseur du microARN ou « pre-miR », puis à la forme mature du microARN ou « miR ».*

*Les termes « micro peptides » et « miPEPs » (microRNA encoded PEPtides) sont équivalents et peuvent être utilisés l'un pour l'autre. Ils définissent un peptide qui est codé par un cadre ouvert de lecture présent sur le transcrit primaire d'un microARN, et qui est capable de moduler l'accumulation dudit microARN. Les micropeptides au sens de la demande* FR 13 60727 *ne doivent être entendus comme étant nécessairement des peptides de petite taille, dans la mesure où « micro » ne correspond pas à la taille du peptide.*

*Compte tenu de la dégénérescence du code génétique, un même micropeptide peut être codé par plusieurs séquences nucléotidiques. De telles séquences nucléotidiques, différentes entre elles d'au moins un nucléotide mais codant un même peptide, sont appelées « séquences dégénérées ».*

*Les termes « cadre ouvert de lecture » ou « ORF » (open reading frame) sont équivalents et peuvent être utilisés l'un pour l'autre. Ils correspondent à une séquence de nucléotides dans une molécule d'ADN ou d'ARN pouvant potentiellement coder un peptide ou une protéine: ledit cadre ouvert de lecture débute par un codon start, suivi d'une série de codons, et se termine par un codon stop.*

*Dans la demande* FR 13 60727*, les ORFs peuvent être dénommés de manière spécifique « miORFs » lorsque ceux-ci sont présents sur les transcrits primaires de microARN.*

*Dans la demande* FR 13 60727*, par « accumulation », on entend la production d'une molécule, telle qu'un microARN ou un micropeptide, dans la cellule.*

*Ainsi, la « modulation » de l'accumulation d'une molécule dans une cellule correspond à une modification de la quantité de cette molécule présente dans la cellule.*

*Dans un mode de réalisation, l'objet de la demande* FR 13 60727 *concerne un procédé de détection et d'identification d'un miPEP tel que défini ci-dessus, dans lequel la modulation de l'accumulation dudit microARN est une diminution ou une augmentation de l'accumulation dudit microARN, en particulier une augmentation.*

*Une « diminution de l'accumulation » correspond à une baisse de la quantité de ladite molécule dans la cellule.*

*A l'inverse, une « augmentation de l'accumulation » correspond à une hausse de la quantité de ladite molécule dans la cellule.*

*Dans un mode de réalisation avantageux, l'objet de la demande* FR 13 60727 *concerne un procédé de détection et d'identification d'un miPEP tel que défini ci-dessus, dans lequel la modulation de l'accumulation dudit microARN est une augmentation de l'accumulation dudit microARN.*

*Dans un mode de réalisation, l'objet de la demande* FR 13 60727 *concerne un procédé de détection et d'identification d'un miPEP tel que défini ci-dessus, dans lequel la présence dudit peptide dans la cellule résulte :*
- *de l'introduction dans la cellule d'un acide nucléique codant ledit peptide, ou*
- *de l'introduction dans la cellule dudit peptide.*

*De manière à caractériser un miPEP, il est nécessaire de disposer d'un modèle cellulaire exprimant un microARN et dans lequel ledit peptide à tester est présent. Pour cela, il est possible d'introduire un peptide dans la cellule, soit en mettant la cellule en contact avec le dit peptide, soit en introduisant dans la cellule un acide nucléique codant ledit peptide, lequel acide nucléique sera alors traduit en peptide à l'intérieur de la cellule.*

*Dans un mode de réalisation, l'objet de la demande* FR 13 60727 *concerne un procédé de détection et d'identification d'un miPEP tel que défini ci-dessus, dans lequel le dit cadre ouvert de lecture de l'étape a) est contenu dans la partie 5' ou 3' dudit transcrit primaire du microARN, de préférence dans la partie 5'.*

*Les parties 5' ou 3' du transcrit primaire du microARN correspondent aux parties terminales de la molécule de l'ARN qui sont clivées au cours de la maturation du microARN. Dans un mode de réalisation, l'objet de la demande* FR 13 60727 *concerne un procédé de détection et d'identification d'un miPEP tel que défini ci-dessus, dans lequel ledit microARN est présent dans une cellule végétale sauvage.*

*Dans la demande* FR 13 60727*, une cellule végétale sauvage correspond à une cellule végétale qui n'a pas été modifiée génétiquement par l'homme.*

*Dans un mode de réalisation, l'objet de la demande* FR 13 60727 *concerne un procédé de détection et d'identification d'un miPEP tel que défini ci-dessus, dans lequel ladite cellule eucaryote déterminée, et ladite cellule eucaryote de même type que la susdite cellule eucaryote déterminée, utilisées à l'étape b, sont des cellules végétales, de préférence des cellules de Medicago truncatula ou d'Arabidopsis thaliana.*

*Dans le procédé de détection et d'identification d'un micropeptide tel que défini ci-dessus, après avoir identifié un ORF susceptible de coder un peptide sur le transcrit primaire d'un microARN, il est nécessaire de disposer d'un modèle cellulaire possédant ledit microARN et ledit peptide, pour pouvoir démontrer un effet éventuel du peptide sur ledit microARN.*

*Deux options sont donc envisageables :*
- *le modèle cellulaire dans lequel a été identifié le miORF et celui dans lequel est mis en évidence l'effet du peptide sur le miR sont identiques, ou*
- *le modèle cellulaire dans lequel a été identifié le miORF et celui dans lequel est mis en évidence l'effet du peptide sur le miR sont différents.*

*Dans la première option, le modèle cellulaire utilisé pour observer un effet du peptide est le même que celui dans lequel le transcrit primaire dudit microARN a été isolé. Dans ce modèle cellulaire, les cellules eucaryotes déterminées contiennent naturellement ledit microARN et seul le peptide à tester doit être introduit dans ces cellules. Dans ce contexte, ledit microARN est qualifié « d'origine endogène » car celui-ci existe naturellement dans les cellules. Néanmoins, dans une cellule, d'autres copies d'un microARN d'origine endogène peuvent être ajoutées, comme par exemple en introduisant dans la cellule un vecteur codant ledit microARN d'origine endogène.*

*Dans la deuxième option, le modèle cellulaire utilisé pour observer un effet du peptide est différent de celui dans lequel le transcrit primaire dudit microARN a été isolé. Dans ce modèle cellulaire, les cellules eucaryotes déterminées ne contiennent ni le microARN, ni le peptide à tester. Ces deux éléments doivent donc être introduits dans ces cellules. Dans ce contexte, ledit microARN est qualifié « d'origine exogène » car celui-ci n'existe pas naturellement dans les cellules.*

*Dans un mode de réalisation, l'objet de la demande* FR 13 60727 *concerne un procédé de détection et d'identification d'un miPEP tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).*

*Dans un mode de réalisation, l'objet de la demande* FR 13 60727 *concerne un procédé de détection et d'identification d'un miPEP tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.*

*Dans un mode de réalisation, l'objet de la demande* FR 13 60727 *concerne un procédé de détection et d'identification d'un miPEP tel que défini ci-dessus, dans lequel l'accumulation dudit microARN est déterminée en mettant en œuvre une RT-PCR quantitative ou un Northern blot.*

*Dans un mode de réalisation, l'objet de la demande* FR 13 60727 *concerne un procédé de détection et d'identification d'un miPEP tel que défini ci-dessus, dans lequel l'accumulation dudit microARN est déterminée en mettant en œuvre une puce à ADN ou à ARN.*

*L'accumulation dudit microARN peut être déterminée à l'aide des techniques de biologie moléculaire permettant le dosage de molécules d'acides nucléiques spécifiques.*

*Dans un autre aspect, l'objet de la demande* FR 13 60727 *concerne également un procédé de détection et d'identification d'un microARN dont la séquence du transcrit primaire contient une séquence nucléotidique codant un miPEP,*
*comprenant :*
- *a) une étape de détection d'un cadre ouvert de lecture de 15 à 303 nucléotides contenu dans la séquence du transcrit primaire dudit microARN, puis*
- *b) une étape de comparaison entre :*
   - *l'accumulation dudit microARN dans une cellule eucaryote déterminée exprimant ledit microARN,*
      *en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et*
   - *l'accumulation dudit microARN dans une cellule eucaryote, de même type que la susdite cellule eucaryote déterminée exprimant ledit microARN,*
   *en absence dudit peptide,*
*dans lequel, une modulation de l'accumulation dudit microARN en présence dudit peptide par rapport à l'accumulation dudit microARN en absence dudit peptide indique l'existence d'un microARN dont le transcrit primaire contient une séquence nucléotidique codant un micropeptide.*

*Dans un mode de réalisation, l'objet de la demande* FR 13 60727 *concerne un procédé de détection et d'identification d'un microARN tel que défini ci-dessus, dans lequel la modulation de l'accumulation dudit microARN est une diminution ou une augmentation de l'accumulation dudit microARN, en particulier une augmentation.*

*Dans un mode de réalisation, l'objet de la demande* FR 13 60727 *concerne un procédé de détection et d'identification d'un microARN tel que défini ci-dessus, dans lequel la présence dudit peptide dans la cellule résulte :*
- *de l'introduction dans la cellule d'un acide nucléique codant ledit peptide, ou*
- *de l'introduction dans la cellule de ledit peptide.*

*Dans un mode de réalisation, l'objet de la demande* FR 13 60727 *concerne un procédé de détection et d'identification d'un microARN tel que défini ci-dessus, dans lequel le dit cadre ouvert de lecture de l'étape a) est contenu dans la partie 5' ou 3' dudit transcrit primaire du microARN, de préférence dans la partie 5'.*

*Dans un mode de réalisation, l'objet de la demande* FR 13 60727 *concerne un procédé de détection et d'identification d'un microARN tel que défini ci-dessus, dans lequel ledit microARN est présent dans une cellule végétale sauvage.*

*Dans un mode de réalisation, l'objet de la demande* FR 13 60727 *concerne un procédé de détection et d'identification d'un microARN tel que défini ci-dessus, dans lequel ladite cellule eucaryote, et ladite cellule eucaryote de même type que la susdite cellule eucaryote déterminée, utilisées à l'étape b) sont des cellules végétales, de préférence des cellules de Medicago truncatula.*

*Dans un mode de réalisation, l'objet de la demande* FR 13 60727 *concerne un procédé de détection et d'identification d'un microARN tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).*

*Dans un mode de réalisation, l'objet de la demande* FR 13 60727 *concerne un procédé de détection et d'identification d'un microARN tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.*

*Dans un mode de réalisation, l'objet de la demande* FR 13 60727 *concerne un procédé de détection et d'identification d'un microARN tel que défini ci-dessus, dans lequel l'accumulation dudit microARN est déterminée en mettant en œuvre une RT-PCR quantitative ou un Northern blot.*

*Dans un mode de réalisation, l'objet de la demande* FR 13 60727 *concerne un procédé de détection et d'identification d'un microARN tel que défini ci-dessus, dans lequel l'accumulation dudit microARN est déterminée en mettant en œuvre une puce à ADN ou à ARN.*

*Dans un autre aspect, l'objet de la demande* FR 13 60727 *concerne un miPEP tel qu'obtenu par la mise en œuvre du procédé tel que défini ci-dessus.*

*Un autre aspect de l'objet de la demande* FR 13 60727 *concerne également un miPEP de 4 à 100 acides aminés, de préférence de 4 à 40 acides aminés, codé par une séquence nucléotidique contenue dans le transcrit primaire d'un microARN, ledit miPEP étant capable de moduler l'accumulation dudit microARN dans une cellule eucaryote.*

*Par ailleurs, il convient de noter que plusieurs miORFS peuvent être identifiés sur le transcrit primaire d'un microARN, indiquant qu'un transcrit primaire de microARN peut potentiellement coder plusieurs miPEPs.*

*Il convient également de noter que l'effet d'un miPEP est généralement spécifique d'un seul microARN, à savoir celui résultant du transcrit primaire codant ledit miPEP.*

*Dans un mode de réalisation, l'objet de la demande* FR 13 60727 *concerne un miPEP tel que défini ci-dessus, ladite séquence nucléotidique étant contenue dans la partie 5' ou 3' dudit transcrit primaire d'un microARN, de préférence dans la partie 5'.*

*Dans un mode de réalisation, l'objet de la demande* FR 13 60727 *concerne un miPEP tel que défini ci-dessus, ladite séquence nucléotidique correspondant au premier cadre ouvert de lecture présent sur ledit transcrit primaire d'un microARN.*

*Dans un mode de réalisation, l'objet de la demande* FR 13 60727 *concerne un miPEP tel que défini ci-dessus, ledit miPEP possédant un point isoélectrique basique, de préférence supérieur à 8.*

*Dans un autre aspect, l'objet de la demande* FR 13 60727 *concerne une molécule d'acide nucléique codant un miPEP tel que défini ci-dessus.*

*Dans autre aspect, l'objet de la demande* FR 13 60727 *concerne un vecteur comprenant au moins une molécule d'acide nucléique tel que définie ci-dessus.*

*Dans un autre aspect, l'objet de la demande* FR 13 60727 *concerne également l'utilisation d'au moins :*
- *un miPEP tel que défini ci-dessus,*
- *un acide nucléique codant ledit miPEP, ou*
- *un vecteur contenant ledit acide nucléique,*
*pour moduler l'expression d'au moins un gène dans une cellule eucaryote déterminée,*
*ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP,*
*l'expression dudit au moins un gène étant régulée par ledit microARN.*

*Dans un autre aspect, l'objet de la demande* FR 13 60727 *concerne également l'utilisation d'au moins :*
- *un miPEP de 4 à 100 acides aminés, de préférence de 4 à 40 acides aminés, codé par une séquence nucléotidique contenue dans le transcrit primaire d'un microARN, ledit miPEP étant capable de moduler l'accumulation dudit microARN dans une cellule eucaryote,*
- *un acide nucléique codant ledit miPEP, ou*
- *un vecteur contenant ledit acide nucléique,*

*pour moduler l'expression d'au moins un gène dans une cellule eucaryote déterminée,*
*ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP,*
*l'expression dudit au moins un gène étant régulée par ledit microARN.*

*L'objet de la demande* FR 13 60727 *repose sur l'observation surprenante faite par les Inventeurs qu'il est possible de moduler l'expression d'un ou plusieurs gènes cibles d'un même microARN en modulant l'accumulation dudit microARN à l'aide d'un miPEP.*

*Dans un mode de réalisation, l'objet de la demande* FR 13 60727 *concerne l'utilisation telle que définie ci-dessus dans laquelle ladite cellule eucaryote déterminée est une cellule végétale.*

*Dans un mode de réalisation, l'objet de la demande* FR 13 60727 *concerne l'utilisation telle que définie ci-dessus dans laquelle ledit microARN et ledit gène sont d'origine endogène dans ladite cellule eucaryote déterminée.*

*Dans un mode de réalisation, l'objet de la demande* FR 13 60727 *concerne l'utilisation telle que définie ci-dessus dans laquelle ledit microARN et ledit gène sont d'origine exogène dans ladite cellule eucaryote déterminée, ladite cellule eucaryote déterminée contenant au moins un vecteur permettant l'expression dudit microARN et dudit gène.*

*Dans la demande* FR 13 60727*, les expressions « d'origine endogène » et « d'origine exogène » sont utilisées pour distinguer lesdits microARNs et*/*ou les gènes d'espèces différentes, étant donné la conservation des séquences entre espèces.*

*Ainsi, le terme « d'origine endogène » indique que le microARN et*/*ou le gène peuvent être présents de manière naturelle dans la cellule en question. De manière artificielle, d'autres copies du microARN et*/*ou du gène d'origine endogène peuvent néanmoins être ajoutées dans la cellule en question, comme par exemple par clonage.*

*A l'inverse, le terme « d'origine exogène » indique que le microARN et*/*ou le gène ne sont jamais présents naturellement dans la cellule en question. Il s'agit d'un microARN et*/*ou d'un gène identifié dans un autre type cellulaire ou dans un organisme d'une autre espèce, ce microARN et*/*ou ce gène sont donc nécessairement introduits artificiellement dans la cellule en question.*

*Dans la demande* FR 13 60727*, une cellule transformée génétiquement peut donc contenir 2 groupes de microARNs et*/*ou de gènes potentiellement proches en termes de séquence, l'un d'origine endogène et l'autre d'origine exogène.*

*Dans un autre aspect, l'objet de la demande* FR 13 60727 *concerne un procédé de modulation de l'expression d'un gène régulée par un microARN dans une cellule eucaryote, comprenant la mise en œuvre d'une étape d'accumulation d'un miPEP dans ladite cellule eucaryote,*
*ledit miPEP ayant :*
- *une taille de 4 à 100 acides aminés, de préférence 4 à 20 acides aminés, et*
- *une séquence peptidique identique à celle codée par une séquence nucléotidique contenue dans le transcrit primaire d'un microARN régulant l'expression dudit gène, et*
- *étant capable de moduler l'accumulation dudit microARN,*
*dans lequel, l'accumulation dudit miPEP dans ladite cellule eucaryote induit une modulation de l'expression dudit gène par rapport à l'expression dudit gène sans accumulation dudit miPEP.*

*Dans un mode de réalisation, l'objet de la demande* FR 13 60727 *concerne un procédé de modulation de l'expression d'un gène tel que défini ci-dessus, dans lequel l'accumulation dudit miPEP dans la cellule résulte :*
- *de l'introduction dans la cellule d'un acide nucléique codant ledit miPEP, ou*
- *de l'introduction dans la cellule dudit miPEP.*

*Dans un mode de réalisation, l'objet de la demande* FR 13 60727 *concerne un procédé de modulation de l'expression d'un gène tel que défini ci-dessus dans lequel ladite cellule eucaryote est une cellule végétale.*

*Dans un mode de réalisation, l'objet de la demande* FR 13 60727 *concerne un procédé de modulation de l'expression d'un gène tel que défini ci-dessus dans lequel ledit microARN et ledit gène sont d'origine endogène dans ladite cellule eucaryote.*

*Dans un mode de réalisation, l'objet de la demande* FR 13 60727 *concerne un procédé de modulation de l'expression d'un gène tel que défini ci-dessus dans lequel ledit microARN et ledit gène sont d'origine exogène dans ladite cellule eucaryote, ladite cellule eucaryote contenant au moins un vecteur permettant l'expression dudit microARN et dudit gène.*

*Dans un autre aspect, l'objet de la demande* FR 13 60727 *concerne une cellule eucaryote modifiée contenant un peptide identique à un miPEP tel que défini ci-dessus, lequel peptide est présent dans ladite cellule eucaryote indépendamment de la transcription du transcrit primaire du microARN portant la séquence nucléotidique codant ledit miPEP.*

*Dans la demande* FR 13 60727*, le terme « cellule eucaryote modifiée » signifie que ladite cellule eucaryote contient un miPEP introduit artificiellement dans la cellule, que ce soit en tant que peptide, ou par l'intermédiaire d'un vecteur codant ledit miPEP.*

*Dans un mode de réalisation, l'objet de la demande* FR 13 60727 *concerne une cellule eucaryote modifiée telle que définie ci-dessus, dans laquelle ledit microARN est d'origine endogène.*

*Dans un autre mode de réalisation, l'objet de la demande* FR 13 60727 *concerne une cellule eucaryote modifiée telle que définie ci-dessus dans laquelle ledit microARN est d'origine exogène, ladite cellule eucaryote modifiée contenant un vecteur permettant l'expression dudit microARN.*

*Dans un mode de réalisation, l'objet de la demande* FR 13 60727 *concerne une cellule eucaryote modifiée telle que définie ci-dessus, ladite cellule étant une cellule végétale.*

*Dans un autre aspect, l'objet de la demande* FR 13 60727 *concerne une plante comprenant au moins une cellule eucaryote modifiée telle que définie ci-dessus.*

*Dans un autre aspect, l'objet de la demande* FR 13 60727 *concerne une composition comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition pesticide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition phytopharmaceutique comprenant au moins* :
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition élicitrice comprenant au moins :*
- *un miPEP tel que défini ci-dessus,*
- *un acide nucléique codant ledit miPEP, ou*
- *un vecteur contenant ledit acide nucléique.*

*Par « composition élicitrice », on désigne une composition capable de conférer à la plante une meilleure aptitude à la symbiose ou une meilleure résistance à différents stress, qu'ils soient de nature thermiques, hydriques ou chimiques.*

*A cet effet, l'objet de la demande* FR 13 60727 *concerne également des compositions agissant sur la croissance (inhibition de la croissance ou au contraire augmentation de la croissance) et la physiologie (meilleure aptitude à mycorhizer, noduler, meilleure tolérance à différents stress) de la plante.*

*Dans un autre aspect, l'objet de la demande* FR 13 60727 *concerne une composition herbicide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition*
*insecticide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne l'utilisation d'une composition telle que définie ci-dessus, en tant qu'herbicide pour éliminer les plantes ou ralentir leur croissance, de préférence en tant qu'herbicide spécifique d'une espèce ou d'un genre de plantes.*

*Dans un autre aspect, l'objet de la demande* FR 13 60727 *concerne l'utilisation d'une composition telle que définie ci-dessus, en tant qu'agent phytopharmaceutique,*
- *pour favoriser la croissance et*/*ou le développement des plantes,*
   *notamment pour la modulation des paramètres physiologiques d'une plante, en particulier la biomasse, la surface foliaire, la floraison, le calibre du fruit, la production et*/*ou la sélection de semence végétales, en particulier pour contrôler la parthénocarpie ou la monoecie d'une plante, ou pour la modification des paramètres physiologiques de semences végétales, en particulier la germination, l'implantation racinaire et la résistance au stress hydrique,*
- *ou pour prévenir ou traiter les maladies des plantes,*
   *en particulier pour favoriser la résistance aux maladies infectieuses.*

*Dans un autre aspect, l'objet de la demande* FR 13 60727 *concerne l'utilisation d'une composition telle que définie ci-dessus, pour moduler les paramètres physiologiques d'une plante, en particulier la biomasse, la surface foliaire, ou le calibre du fruit.*

*Dans un mode de réalisation, l'objet de la demande* FR 13 60727 *concerne l'utilisation d'une composition telle que définie ci-dessus, pour l'éclaircissage de vergers afin d'augmenter le calibre des fruits.*

*Dans un mode de réalisation, l'objet de la demande* FR 13 60727 *concerne l'utilisation d'une composition telle que définie ci-dessus, pour la production et*/*ou la sélection de semences végétales, ladite composition étant utilisée pour contrôler la parthénocarpie ou la monoecie d'une plante.*

*Dans un mode de réalisation, l'objet de la demande* FR 13 60727 *concerne l'utilisation d'une composition telle que définie ci-dessus, ladite composition étant administrée à ladite plante par la voie foliaire ou par la voie racinaire.*

*Dans un mode de réalisation, l'objet de la demande* FR 13 60727 *concerne l'utilisation d'une composition telle que définie ci-dessus, pour la production et*/*ou la sélection de semences végétales.*

*Dans un mode de réalisation, l'objet de la demande* FR 13 60727 *concerne l'utilisation d'une composition telle que définie ci-dessus, dans laquelle ladite composition est utilisée pour modifier les paramètres physiologiques desdites semences végétales, en particulier l'implantation racinaire, la germination et la résistance au stress hydrique.*

*Dans un mode de réalisation, l'objet de la demande* FR 13 60727 *concerne l'utilisation d'une composition telle que définie ci-dessus, dans laquelle ladite composition est appliquée par enrobage ou pelliculage sur lesdites semences végétales.*

*Dans un autre aspect, l'objet de la demande* FR 13 60727 *concerne l'utilisation d'une composition telle que définie ci-dessus, en tant que pesticide, pour éliminer les organismes nuisibles des plantes ou susceptibles d'être classés comme tels,*
*notamment en tant qu'insecticide, arachnicide, limacide ou rodonticide.*

*Dans un mode de réalisation, l'objet de la demande* FR 13 60727 *concerne l'utilisation d'une composition telle que définie ci-dessus, en tant qu'insecticide.*

*Dans un mode de réalisation, l'objet de la demande* FR 13 60727 *concerne l'utilisation d'une composition telle que définie ci-dessus, pour éliminer les insectes ravageurs.*

*Dans un mode de réalisation, l'objet de la demande* FR 13 60727 *concerne l'utilisation d'une composition telle que définie ci-dessus, pour éliminer les espèces animales classées nuisibles ou susceptibles d'être classées comme telles, en particulier les muridae, notamment le rat.*

*Dans un autre aspect, l'objet de la demande* FR 13 60727 *concerne l'utilisation d'une composition telle que définie ci-dessus, dans laquelle ladite composition est appliquée sur une plante pour la protéger d'insectes ravageurs.*

### LEGENDES DES FIGURES

**FIGURE 1****. Effets d'un traitement avec le miPEP172c sur l'expression du miR172c chez *Glycine max***
   L'axe des ordonnées indique l'expression relative du miR172c chez une plante contrôle (colonne de gauche) ou chez une plante arrosée avec le miPEP172c à 0,1 µM (colonne de droite). La barre d'erreur correspond à l'erreur standard à la moyenne (nombre d'individus = 9).
**FIGURE 2****. Effets du miPEP172c sur le nombre de nodules chez *Glycine max***
   L'axe des ordonnées indique le nombre de nodules par gramme de poids frais de racine de *Glycine max* traitée par un solvant (contrôle, barre de gauche) ou par un solvant contenant 0,1 µM de miPEP172c (miPEP172c, barre de droite).
   La barre d'erreur correspond à l'erreur standard à la moyenne (nombre d'individus = 9). Cette expérience a été répétée de manière indépendante et a abouti à des résultats similaires.

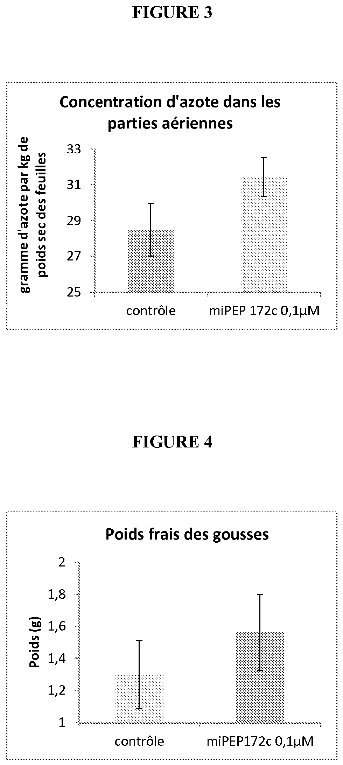

**FIGURE 3****. Effets du miPEP172c sur la concentration d'azote dans les parties aériennes chez *Glycine max***
   L'axe des ordonnées indique la concentration d'azote par poids sec de feuilles de *Glycine max* (en g/kg), traitée par un solvant (contrôle, barre de gauche) ou par un solvant contenant 0,1 µM de miPEP172c (miPEP172c, barre de droite).
   La barre d'erreur correspond à l'erreur standard à la moyenne (nombre d'individus = 9).
**FIGURE 4****. Effets du miPEP172c sur le poids frais des gousses chez *Glycine max***
   L'axe des ordonnées indique le poids frais sec des gousses chez *Glycine max* (en g), traitée par un solvant (contrôle, barre de gauche) ou par un solvant contenant 0,1 µM de miPEP 172c (miPEP172c, barre de droite).
   La barre d'erreur correspond à l'erreur standard à la moyenne (nombre d'individus = 9).

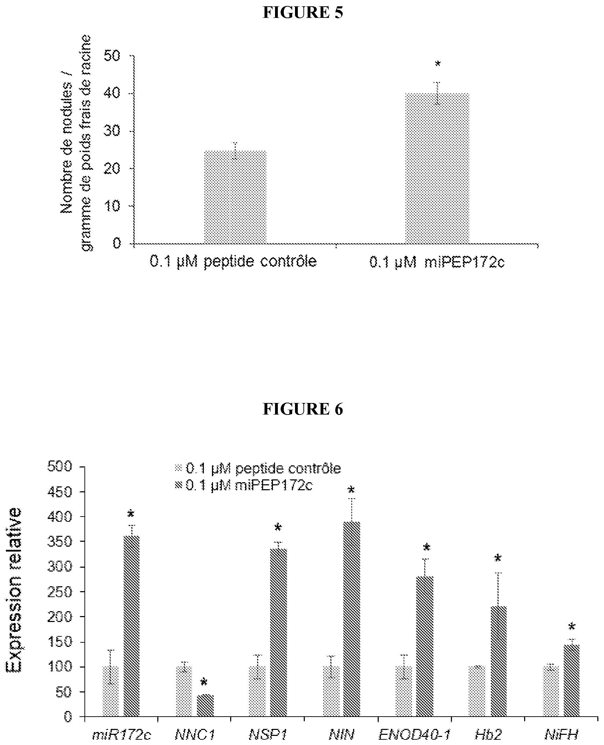

**FIGURE 5****. Effets du miPEP172c sur le nombre de nodules chez *Glycine max***
   L'axe des ordonnées indique le nombre de nodules par gramme de poids frais de racine de *Glycine max* traitée par un solvant contenant un peptide contrôle à 0,01 µM (barre de gauche) ou par un solvant contenant 0,1 µM de miPEP 172c (barre de droite).
   La barre d'erreur correspond à l'erreur standard à la moyenne (nombre d'individus = 12). La présence d'un astérisque indique une différence d'expression significative entre les deux conditions testées.
**FIGURE 6****. Effets du miPEP172c sur l'expression du miR172c et sur l'expression des gènes *NNC1, NSP1, NIN, ENOD40-1, Hb2* et *nitH* chez *Glycine max.***
   L'axe des ordonnées indique l'expression relative du miR172c et des gènes *NNC1, NSP1, NIN, ENOD40-1, Hb2 et nifH* chez une plante contrôle arrosée avec un solvant contenant un peptide contrôle à 0,01 µM (colonnes blanches) ou chez une plante arrosée un solvant contenant le miPEP172c à 0,01 µM (colonnes noires). La barre d'erreur correspond à l'erreur standard à la moyenne (nombre d'individus = 6). La présence d'un astérisque indique une différence d'expression significative entre les deux conditions testées.

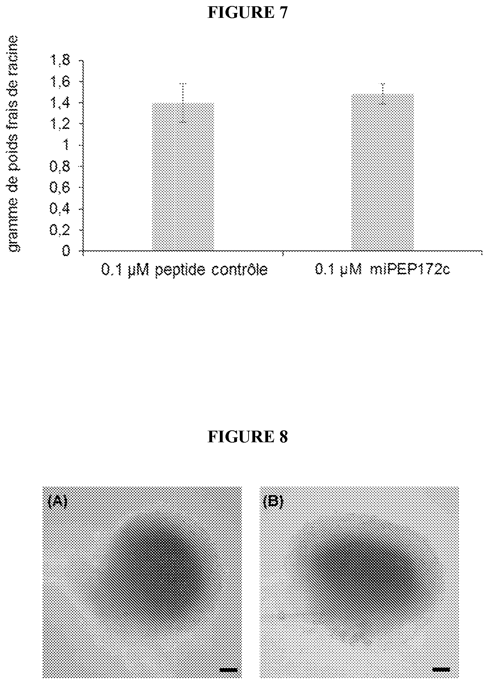

**FIGURE 7****. Effets du miPEP172c sur la masse de racines chez *Glycine max.***
   L'axe des ordonnées indique le poids frais de racine en grammes chez une plante contrôle arrosée avec un solvant contenant un peptide contrôle à 0,01 µM (colonne de gauche) ou chez une plante arrosée un solvant contenant le miPEP172c à 0,01 µM (colonne de droite) (nombre d'individus = 6).
**FIGURE 8****. Expression du gène *NifD.***
   Les photographies représentent l'expression de *nifD* révélée par des fusions nifD-LacZ dans des nodules traités par le peptide contrôle (A) ou par le miPEP172c (B).

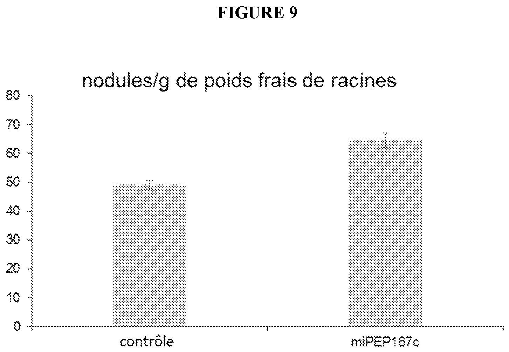

**FIGURE 9****. Effets du miPEP167c sur le nombre de nodules chez *Glycine max***
   L'axe des ordonnées indique le nombre de nodules par gramme de poids frais de racine de *Glycine max* traitée par un solvant (contrôle, barre de gauche) ou par un solvant contenant 0,1 µM de miPEP167c (miPEP167c, barre de droite).
   La barre d'erreur correspond à l'erreur standard à la moyenne (nombre d'individus = 12).

### EXEMPLES

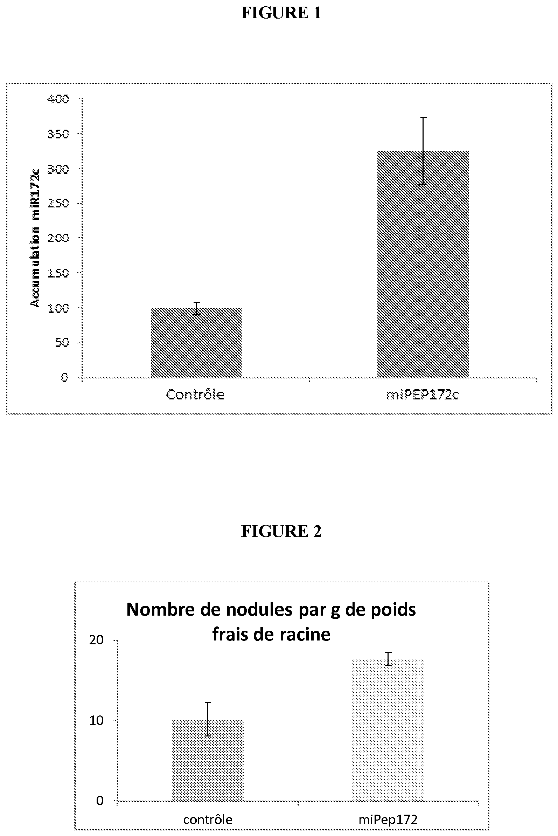

Le miPEP172c augmente l'expression du miR172c (Figures 1 et 6). L'effet du miPEP172c est agoniste à celui du miR172c, en réduisant l'expression du gène *NNC1* (facteur de transcription répressif) et en entraînant une augmentation de l'expression des gènes *NSP1, NIN, ENOD40-1, Hb2 et NifH* impliqués dans la nodulation (Figure 6).

L'arrosage de plantes de soja (*Glycine max*) par de faibles concentrations de miPEP172c (0,1 µM) spécifique du miR172c augmente significativement le nombre de nodules (Figures 2 et 5) et la teneur en azote des parties aériennes (Figure 3), ainsi que le poids frais des gousses (Figure 4). Aucun effet n'est observé sur le développement des racines (Figure 7). Une augmentation du nombre de nodules inactifs intervient parfois comme un mécanisme de compensation en réponse à une fixation réduite de l'azote. L'analyse de l'expression du gène *NifH* par RT-qPCR (Figure 6) et l'observation des fusions *nifD::LacZ* (Figure 8) indiquent toutefois une fixation efficace de l'azote chez les plantes traitées par le miPEP172c.

L'ensemble de ces résultats indique que le traitement avec le miPEP172c mime les effets d'une surexpression du miR172c, tant au niveau moléculaire que phénotypique. L'arrosage de plantes de soja (*Glycine max*) par de faibles concentrations de miPEP167c (0,1 µM) spécifique du miR167c augmente également de manière significative le nombre de nodules (Figure 9).

### Matériel et méthodes

### • Mesure de l'expression du miR172c et des gènes NNC1, NSP1, NIN et ENOD40-1 chez Glycine max

L'ARN total des racines de *soja* a été extrait en utilisant le kit d'extraction RNeasy Plant Mini Kit (Qiagen). La transcription inverse a été réalisée en utilisant la transcriptase inverse SuperScript II (Invitrogen) à partir de 500 ng d'ARN total. Trois répétitions (n=3) ont été réalisées avec deux répétitions techniques chacune. Chaque expérience a été répétée de deux à trois fois. Les amplifications par qPCR ont été réalisées en utilisant un thermocycleur LightCycler 480 System (Roche Diagnostics) selon la méthode décrite dans Lauressergues et al. (Plant J., 72(3):512-22, 2012). *ELF1b* a été utilisé comme gène de ménage pour normaliser les analyses qRT-PCR. Les amorces utilisées pour l'amplification des gènes *ELF1b, NNC1, NSP1, NIN, ENOD40-1, Hb2* et *nifH* sont décrites dans les articles de Wang et al. (Soybean miR172c targets the repressive AP2 transcription factor NNC1 to activate ENOD40 expression and regulate nodule initiation, Plant cell 26(12) : 4782-4801, 2014; MicroRNA167-directed regulation of the auxin response factors GmARF8a and GmARF8b is required for soybean nodulation and lateral root development, Plant Physiol 168(3): 984-999, 2015).

### • Germination des graines de G. max :

Après stérilisation des graines pendant 3 min dans de la javel dilué au ¼, puis rinçage avec de l'eau stérile, les graines sont incubées 2 heures dans de l'eau. Enfin, les graines sont placées dans une boite de pétri avec du papier humide à 28°C. Une fois les graines germées elles sont placées dans un petit pot contenant du milieu oil dry^{®} sous cloche. Après quelques jours de croissance, les plantes dont le développement est uniforme sont sélectionnées pour l'expérience et transférées dans un pot plus grand.

### • Inoculation des plantules germées avec Bradirhizobium japonicum :

Les bactéries sont mises en culture dans du milieu liquide Campbel (K₂PO₄ : 0,5g/l, MgSO₄ 7H₂O : 0,2g/l, NaCl : 0,1g/l, Mannitol: 10g/l, Extrait de levure: 2,5g/l, Casamino acid: 0,5g/l) (5 ml) à 28°C, puis transférées dans un Erlenmeyer contenant 100ml de milieu Campbel. Après quelques jours de culture, lorsque la DO est 0,3 à 595 nm, les 100 ml de culture de *B. japonicum* ont été centrifugés pendant 30 min à 4000rpm. Le surnageant a été jeté et le culot resuspendu dans du milieu de culture « caisson » (Lullien et al., Plant gene expression in effective and ineffective root nodules of alfalfa (Medicago sativa). Plant Mol Biol 9: 469-478, 1987) pour atteindre la DO de 0,05 à 595 nm. Chaque plante est inoculée avec 20ml de cette solution. Les plantes sont régulièrement arrosées avec du milieu « caisson ».

### • Traitements des plantes avec les miPEP :

Le traitement s'effectue par arrosage des plantes avec la solution concentrée de miPEP ou l'équivalent de solution de solvant pour le traitement contrôle. Un traitement contrôle est également réalisé avec une solution de solvant contenant un peptide contrôle (SEQ ID NO : 5, VLWCSHCMGFLWPTYT). Le peptide contrôle et le miPEP172c ont le même contenu en acides aminés, mais des séquences différentes. Les traitements sont réalisés tous les deux jours.

## Revendications

1. Utilisation d'un miPEP introduit par voie exogène dans une plante pour favoriser la nodulation, entre ladite plante et une bactérie, ledit miPEP étant également naturellement présent dans ladite plante,
ledit miPEP introduit par voie exogène étant soit un peptide produit hors de la plante, soit un peptide produit dans la plante suite à l'introduction non naturelle d'un acide nucléique codant ledit miPEP dans ladite plante,
ledit miPEP introduit par voie exogène étant un peptide comprenant, ou consistant, en une séquence identique à celle dudit miPEP naturellement présent,
lequel miPEP naturellement présent est un peptide de 3 à 100 acides aminés dont la séquence est codée par un cadre ouvert de lecture situé en 5' sur le transcrit primaire d'un miR,
ledit miPEP étant capable de moduler l'accumulation dudit miR dans ladite plante, lequel miR régule l'expression d'au moins un gène impliqué dans la nodulation chez ladite plante, la somme de la quantité dudit miPEP introduit par voie exogène et de celle dudit miPEP naturellement présent étant strictement supérieure à la quantité dudit miPEP naturellement présent,
ledit miPEP étant le miPEP172c possédant une séquence d'acides aminés présentant au moins 80% d'identité avec la séquence SEQ ID NO : 2,
en particulier le miPEP172c possédant une séquence d'acides aminés consistant en la séquence SEQ ID NO : 2,
de préférence, ledit miR étant le miR172c possédant en particulier une séquence nucléotidique consistant en SEQ ID NO : 1.

2. Utilisation selon la revendication 1, dans laquelle ladite plante est :
- une plante légumineuse, notamment choisie parmi : le Lotus (*Lotus sp.)* le soja (*Glycine max*), l'arachide (*Arachis hypogaea*), le haricot (*Phaseolus vulgaris*), le pois (*Pisum sativum*), la lentille (*Lens culinaris),* le pois chiche (*Cicer arietinum),* la fève et la féverolle (*Vicia faba*), les vesces (*Vicia* sp.), les gesses (*Lathyrus* sp.), la luzerne (*Medicago* sp.), le trèfle (*Trifolium* sp.), le lupin (*Lupinus* sp.), le haricot mungo (*Vigna radiata*), la réglisse (*Glycyrrhiza glabra*), le palissandre (*Dalbergia*), le lotier corniculé (*Lotus corniculatus*), le sainfoin (*Onobrychis viciifolia*), le rooibos *(Aspalathus linearis),* le fenugrec (*Trigonella foenum-graecum*), ou
- la betterave sucrée (*Beta vulgaris*).

3. Utilisation selon l'une quelconque des revendications précédentes, dans laquelle ladite bactérie est une bactérie de la famille des *Rhizobiaceae,* notamment choisie parmi les genres *Rhizobium, Sinorhizobium, Mesorhizobium, Bradyrhizobium* ou *Azorhizobium.*

4. Procédé pour favoriser la nodulation entre une plante et une bactérie, comprenant une étape d'introduction artificielle d'un miPEP dans une plante par voie exogène, ledit miPEP étant également présent naturellement dans ladite plante,
ledit miPEP introduit par voie exogène étant soit un peptide produit hors de la plante, soit un peptide produit dans la plante suite à l'introduction non naturelle d'un acide nucléique codant ledit miPEP dans ladite plante,
ledit miPEP introduit par voie exogène étant un peptide de 3 à 100 acides aminés dont la séquence comprend ou consiste en une séquence identique à celle dudit miPEP naturellement présent, laquelle séquence du miPEP naturellement présent est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante,
la somme de la quantité dudit miPEP introduit par voie exogène et de celle dudit miPEP naturellement présent étant strictement supérieure à la quantité dudit miPEP naturellement présent,
ledit miPEP étant le miPEP172c possédant une séquence d'acides aminés présentant au moins 80% d'identité avec la séquence SEQ ID NO : 2, en particulier le miPEP172c possédant une séquence d'acides aminés consistant en la séquence SEQ ID NO : 2,
de préférence, ledit miR étant le miR172c possédant en particulier une séquence nucléotidique consistant en SEQ ID NO : 1,
ladite bactérie étant notamment de la famille des *Rhizobiaceae,*
ladite plante étant notamment une plante légumineuse.

5. Procédé selon la revendication 4, pour favoriser la nodulation entre une plante *Glycine max* et une bactérie *Bradirhizobium japonicum,* dans lequel le miPEP172c est introduit par voie exogène dans ladite plante *Glycine max,* ledit miPEP172c étant également naturellement présent dans ladite plante *Glycine max,*
ledit miPEP172c introduit par voie exogène étant un peptide comprenant ou consistant en une séquence identique à celle dudit miPEP172c naturellement présent, lequel miPEP172c naturellement présent est un peptide de 3 à 100 acides aminés dont la séquence est codée par un cadre ouvert de lecture situé en 5' sur le transcrit primaire du miR172c,
ledit miPEP172c étant capable de moduler l'accumulation dudit miR172c, lequel miR172c régule l'expression d'au moins un gène impliqué dans la nodulation chez *Glycine max,*
la somme de la quantité dudit miPEP172c introduit par voie exogène et de celle dudit miPEP172c naturellement présent étant strictement supérieure à la quantité dudit miPEP172c naturellement présent.

6. Procédé selon l'une quelconque des revendications 4 ou 5, dans lequel ledit miPEP est introduit dans la plante :
- par voie externe, de préférence par arrosage, par pulvérisation ou par l'ajout d'un engrais, d'un terreau, d'un substrat de culture ou à l'aide d'un support en contact avec la plante, ledit miPEP étant notamment administré à la plante sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP,
- par voie externe, de préférence par arrosage, par pulvérisation ou par l'ajout d'un engrais, d'un terreau, d'un substrat de culture ou à l'aide d'un support en contact avec une graine ou une semence, ledit miPEP étant notamment administré à la graine ou à la semence sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP, ou
- par le biais d'un acide nucléique codant ledit miPEP, ledit acide nucléique étant introduit par voie exogène dans la plante, mais ledit acide nucléique ne comprenant pas la séquence complète du miR correspondant.

7. Procédé de production d'une plante transgénique comprenant :
**a)** une étape d'introduction d'un acide nucléique codant un miPEP de 3 à 100 acides aminés dans une plante, mais ledit acide nucléique ne comprenant pas la séquence complète du miR correspondant, ou dans au moins une cellule de ladite plante, dans des conditions permettant l'expression dudit miPEP,
ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans la nodulation, et
**b)** une étape de culture de la plante, ou d'au moins une cellule de ladite plante, obtenues à l'étape a) dans des conditions permettant l'obtention d'une plante transgénique,
ledit miPEP étant le miPEP172c, ledit miPEP172c possédant une séquence d'acides aminés présentant au moins 80% d'identité avec la séquence SEQ ID NO : 2,
en particulier ledit miPEP172c possédant une séquence d'acides aminés consistant en la séquence SEQ ID NO : 2,
ledit miR étant notamment le miR172c, ledit miR172c possédant en particulier une séquence nucléotidique consistant en SEQ ID NO : 1.

8. Plante transgénique telle qu'obtenue par le procédé tel que défini selon la revendication 7.

9. Peptide ayant une séquence d'acides aminés présentant au moins 80% d'identité, de préférence au moins 90 % d'identité, avec la séquence d'acides aminés SEQ ID NO : 2, de préférence ledit peptide comprenant ou consistant en la séquence d'acides aminés SEQ ID NO : 2,
ledit peptide étant capable de moduler 1 accumulation du miR172c.

10. Composition comprenant :
- le miPEP172c en tant que substance active, ledit miPEP172c comprenant ou consistant en une séquence d'acides aminés présentant au moins 80% d'identité avec la séquence SEQ ID NO : 2,
en particulier ledit miPEP172c comprenant ou consistant en une séquence d'acides aminés consistant en la séquence SEQ ID NO : 2,
ledit miPEP172c étant notamment à une concentration de 10⁻⁹ M à 10⁻⁴ M, en particulier 10⁻⁹, 10⁻⁸, 10⁻⁷, 10⁻⁶, 10⁻⁵ ou 10⁻⁴ M, ou
en particulier, ladite composition comprenant de plus un excipient, un diluant ou un solvant, en particulier, ladite composition étant formulée de façon à former un enrobé.

11. Composition comprenant en combinaison une quantité de semences d'une plante et une quantité d'un peptide synthétique dont la séquence comprend ou consiste en une séquence identique à celle d'un miPEP naturellement présent chez ladite plante,
ledit peptide synthétique ayant une séquence comprenant ou consistant en une séquence identique à celle du miPEP172c, ledit miPEP172c possédant une séquence d'acides aminés présentant au moins 80% d'identité avec la séquence SEQ ID NO : 2,
en particulier ledit miPEP172c possédant une séquence d'acides aminés consistant en la séquence SEQ ID NO : 2,
en particulier, ladite composition étant formulée de façon à former une semence enrobée.

12. Semence enrobée avec une composition comprenant un miPEP capable de moduler l'accumulation d'un miR chez la plante issue de la semence, lequel miR régule l'expression d'au moins un gène impliqué dans la nodulation chez ladite plante,
ledit miPEP étant le miPEP172c, ledit miPEP172c possédant une séquence d'acides aminés présentant au moins 80% d'identité avec la séquence SEQ ID NO : 2,
en particulier ledit miPEP172c possédant une séquence d'acides aminés consistant en la séquence SEQ ID NO : 2.

13. Utilisation d'un miPEP pour la culture de bactéries, en particulier de bactéries de la famille des *Rhizobiaceae,* ladite utilisation comprenant une étape de mise en contact desdites bactéries avec :
- un mélange comprenant une plante ou une partie de plante, en particulier une culture de racine, et un peptide dont la séquence comprend ou consiste en une séquence identique à celle d'un miPEP naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante, ou
- avec une plante transgénique telle que définie précédemment,
la plante, la partie de plante, et la plante transgénique étant aptes à la nodulation avec ladite bactérie,
ledit miPEP étant le miPEP172c, ledit miPEP172c possédant une séquence d'acides aminés présentant au moins 80% d'identité avec la séquence SEQ ID NO : 2,
en particulier ledit miPEP172c possédant une séquence d'acides aminés consistant en la séquence SEQ ID NO : 2.

14. Utilisation d'un miPEP pour produire de l'inoculum de bactéries, en particulier de l'inoculum de bactéries de la famille des *Rhizobiaceae,* ladite utilisation comprenant :
- une étape de co-culture de bactéries avec une matière végétale vivante, dite plante hôte, correspondant au moins en partie, à une partie constitutive de racine d'une plante apte à former une nodulation avec lesdites bactéries, et
- une étape de mise en contact d'une quantité d'un peptide avec la susdite co-culture,
ledit peptide ayant une séquence comprenant ou consistant en une séquence identique à celle d'un miPEP naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante,
ledit miPEP étant le miPEP172c, ledit miPEP172c possédant une séquence d'acides aminés présentant au moins 80% d'identité avec la séquence SEQ ID NO : 2,
en particulier ledit miPEP172c possédant une séquence d'acides aminés consistant en la séquence SEQ ID NO : 2.

15. Inoculum de bactéries, en particulier un inoculum de bactéries de la famille des *Rhizobiaceae,* adapté à l'inoculation d'une plante hôte, comprenant au moins : une bactérie et un peptide dont la séquence comprend ou consiste en une séquence identique à celle d'un miPEP naturellement présent dans la plante hôte,
ledit miPEP naturellement présent dans la plante hôte étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la nodulation chez ladite plante hôte,
ledit miPEP étant le miPEP172c, ledit miPEP172c possédant une séquence d'acides aminés présentant au moins 80% d'identité avec la séquence SEQ ID NO : 2,
en particulier ledit miPEP172c possédant une séquence d'acides aminés consistant en la séquence SEQ ID NO : 2.

## Patentansprüche

1. Verwendung eines exogen in eine Pflanze eingeführten miPEPs zur Förderung der Nodulation zwischen der Pflanze und einem Bakterium, wobei das miPEP auch natürlich in der Pflanze vorkommt,
wobei das exogen eingeführte miPEP entweder ein außerhalb der Pflanze produziertes Peptid oder ein Peptid ist, das in der Pflanze infolge der nicht natürlichen Einführung einer für das miPEP kodierenden Nukleinsäure in die Pflanze produziert wird,
wobei das exogen eingeführte miPEP ein Peptid ist, das eine Sequenz umfasst oder daraus besteht, die mit derjenigen des natürlich vorkommenden miPEP identisch ist,
wobei das natürlich vorkommende miPEP ein Peptid mit 3 bis 100 Aminosäuren ist, dessen Sequenz von einem offenen Leserahmen kodiert wird, der sich in 5' auf dem primären Transkript einer miR befindet,
wobei das miPEP in der Lage ist, die Akkumulation der miR in der Pflanze zu modulieren, wobei die miR die Expression von mindestens einem Gen reguliert, das an der Nodulation in der Pflanze beteiligt ist, wobei die Summe der Menge des exogen eingeführten miPEP und des natürlich vorkommenden miPEP strikt größer ist als die Menge des natürlich vorkommenden miPEP,
wobei das miPEP das miPEP172c ist, das eine Aminosäuresequenz besitzt, die mindestens 80 % Identität mit der Sequenz SEQ ID NO: 2 aufweist,
wobei das miPEP172c insbesondere eine Aminosäuresequenz besitzt, die aus der Sequenz SEQ ID NO: 2 besteht,
wobei die miR vorzugsweise die miR172c ist, die insbesondere eine Nukleotidsequenz besitzt, die aus SEQ ID NO: 1 besteht.

2. Verwendung nach Anspruch 1, wobei die Pflanze
- eine Leguminosenpflanze ist, insbesondere ausgewählt aus: Lotus (*Lotus sp*.), Soja (*Glycine max*), Erdnuss (*Arachis hypogaea*), Bohne (*Phaseolus vulgaris*)*,* Erbse (*Pisum sativum*)*,* Linse (*Lens culinaris*)*,* Kichererbse (*Cicer arietinum*), Ackerbohne und Saubohne (*Vicia faba*)*,* Wicken (*Vicia sp*.), Platterbsen (*Lathyrus sp*.), Luzerne (*Medicago sp.*), Klee (*Trifolium sp*.), Lupine (*Lupinus sp*.), *Mungobohne* (*Vigna radiata*), Echtem Süßholz (*Glycyrrhiza glabra*), Palisander (*Dalbergia*), Gewöhnlichem Hornklee (*Lotus corniculatus*), Saat-Esparsette (*Onobrychis viciifolia*)*,* Rooibos (*Aspalathus linearis*)*,* Bockshornklee (*Trigonellafoenum-graecum*) oder
- Zuckerrübe (*Beta vulgaris*).

3. Verwendung nach einem der vorhergehenden Ansprüche, wobei das Bakterium ein Bakterium aus der Familie der *Rhizobiaceae* ist, insbesondere ausgewählt aus den Gattungen *Rhizobium, Sinorhizobium, Mesorhizobium, Bradyrhizobium* oder *Azorhizobium.*

4. Verfahren zur Förderung der Nodulation zwischen einer Pflanze und einem Bakterium, umfassend einen Schritt zur exogenen künstlichen Einführung eines miPEP in eine Pflanze, wobei das miPEP auch natürlich in der Pflanze vorkommt,
wobei das exogen eingeführte miPEP entweder ein außerhalb der Pflanze produziertes Peptid oder ein Peptid ist, das in der Pflanze infolge der nicht natürlichen Einführung einer für das miPEP kodierenden Nukleinsäure in die Pflanze produziert wird,
wobei das exogen eingeführte miPEP ein Peptid mit 3 bis 100 Aminosäuren ist, dessen Sequenz eine Sequenz umfasst oder aus einer Sequenz besteht, die mit der des natürlich vorkommenden miPEP identisch ist, wobei die Sequenz des natürlich vorkommenden miPEP durch einen offenen Leserahmen kodiert wird, der sich in 5' auf dem primären Transkript einer miR befindet, wobei das miPEP in der Lage ist, die Akkumulation der miR zu modulieren, wobei die miR die Expression mindestens eines Gens reguliert, das an der Nodulation in der Pflanze beteiligt ist,
wobei die Summe der Menge des exogen eingeführten miPEPs und des natürlich vorkommenden miPEPs strikt größer ist als die Menge des natürlich vorkommenden miPEPs,
wobei das miPEP das miPEP172c ist, das eine Aminosäuresequenz besitzt, die mindestens 80 % Identität mit der Sequenz SEQ ID NO: 2 aufweist, wobei das miPEP172c insbesondere eine Aminosäuresequenz besitzt, die aus der Sequenz SEQ ID NO: 2 besteht,
wobei die miR vorzugsweise die miR172c ist, die insbesondere eine Nukleotidsequenz besitzt, die aus SEQ ID NO: 1 besteht,
wobei das Bakterium insbesondere aus der Familie der *Rhizobiaceae* stammt,
wobei die Pflanze insbesondere eine Leguminosenpflanze ist.

5. Verfahren nach Anspruch 4 zur Förderung der Nodulation zwischen einer *Glycine-max-Pflanze* und einem Bakterium *Bradirhizobium japonicum,* wobei das miPEP172c exogen in die *Glycine-max-Pflanze* eingeführt wird, wobei das miPEP172c auch natürlich in der *Glycine-max-Pflanze* vorkommt,
wobei das exogen eingeführte miPEP172c ein Peptid ist, das eine Sequenz umfasst oder aus einer Sequenz besteht, die mit der des natürlich vorkommenden miPEP172c identisch ist, wobei das natürlich vorkommende miPEP172c ein Peptid mit 3 bis 100 Aminosäuren ist, dessen Sequenz durch einen offenen Leserahmen kodiert wird, der sich in 5' auf dem primären miR172c-Transkript befindet,
wobei das miPEP172c in der Lage ist, die Akkumulation der miR172c zu modulieren, wobei die miR172c die Expression von mindestens einem Gen reguliert, das an der Nodulation in *Glycine max* beteiligt ist, wobei die Summe der Menge des exogen eingeführten miPEP172c und des natürlich vorkommenden miPEP172c strikt größer ist als die Menge des natürlich vorkommenden miPEP172c.

6. Verfahren nach einem der Ansprüche 4 oder 5, wobei das miPEP folgendermaßen in die Pflanze eingeführt wird:
- von außen, vorzugsweise durch Bewässern, Besprühen oder Zugeben eines Düngers, einer Blumenerde, eines Kultursubstrats oder mithilfe eines Trägers, der mit der Pflanze in Kontakt kommt, wobei das miPEP insbesondere in Form einer Zusammensetzung an die Pflanze appliziert wird, die 10⁻⁹ M bis 10⁻⁴ M des miPEP, insbesondere 10⁻⁹, 10⁻⁸, 10⁻⁷, 10⁻⁶, 10⁻⁵ oder 10⁻⁴ M des miPEP umfasst,
- von außen, vorzugsweise durch Bewässern, Besprühen oder Zugeben eines Düngers, einer Blumenerde, eines Kultursubstrats oder mithilfe eines Trägers, der mit einem Samenkorn oder einem Saatgut in Kontakt kommt, wobei das miPEP dem Samenkorn oder dem Saatgut insbesondere in Form einer Zusammensetzung appliziert wird, die 10⁻⁹ M bis 10⁻⁴ M des miPEPs, insbesondere 10⁻⁹, 10⁻⁸, 10⁻⁷, 10⁻⁶, 10⁻⁵ oder 10⁻⁴ M des miPEPs, umfasst, oder
- mittels einer Nukleinsäure, die für das miPEP kodiert, wobei die Nukleinsäure exogen in die Pflanze eingeführt wird, die Nukleinsäure jedoch nicht die vollständige Sequenz der entsprechenden miR umfasst.

7. Verfahren zur Produktion einer transgenen Pflanze, umfassend:
a) einen Schritt zum Einführen einer Nukleinsäure, die ein miPEP von 3 bis 100 Aminosäuren kodiert, in eine Pflanze, wobei die Nukleinsäure jedoch nicht die vollständige Sequenz der entsprechenden miR umfasst, oder in mindestens eine Zelle der Pflanze unter Bedingungen, die die Expression des miPEP ermöglichen,
wobei das miPEP auch natürlich in der Pflanze vorkommt, wobei das natürlich vorkommende miPEP ein Peptid ist, dessen Sequenz von einem offenen Leserahmen kodiert wird, der sich in 5' auf dem primären Transkript einer miR befindet, wobei das miPEP in der Lage ist, die Akkumulation der miR in der Pflanze zu modulieren, wobei die miR die Expression von mindestens einem Gen reguliert, das an der Nodulation beteiligt ist, und
b) einen Schritt zum Kultivieren der in Schritt a) erhaltenen Pflanze oder mindestens einer Zelle der Pflanze unter Bedingungen, die den Erhalt einer transgenen Pflanze ermöglichen,
wobei das miPEP das miPEP172c ist, wobei das miPEP172c eine Aminosäuresequenz besitzt, die mindestens 80 % Identität mit der Sequenz SEQ ID NO: 2 aufweist,
wobei das miPEP172c insbesondere eine Aminosäuresequenz besitzt, die aus der Sequenz SEQ ID NO: 2 besteht,
wobei die miR insbesondere die miR172c ist, wobei die miR172c insbesondere eine Nukleotidsequenz besitzt, die aus SEQ ID NO: 1 besteht.

8. Transgene Pflanze, wie sie durch das Verfahren nach Anspruch 7 erhalten wurde.

9. Peptid mit einer Aminosäuresequenz, die mindestens 80 % Identität, vorzugsweise mindestens 90 % Identität, mit der Aminosäuresequenz SEQ ID NO: 2 aufweist,
wobei das Peptid vorzugsweise die Aminosäuresequenz SEQ ID NO: 2 umfasst oder daraus besteht,
wobei das Peptid in der Lage ist, die Akkumulation von miR172c zu modulieren.

10. Zusammensetzung, umfassend:
- das miPEP172c als Wirkstoff, wobei das miPEP172c eine Aminosäuresequenz umfasst oder daraus besteht, die mindestens 80 % Identität mit der Sequenz SEQ ID NO: 2 aufweist,
wobei das miPEP172c insbesondere eine Aminosäuresequenz umfasst oder daraus besteht, die aus der Sequenz SEQ ID NO: 2 besteht,
wobei das miPEP172c insbesondere in einer Konzentration von 10⁻⁹ M bis 10⁻⁴ M, insbesondere 10⁻⁹, 10⁻⁸, 10⁻⁷, 10⁻⁶, 10⁻⁵ oder 10⁻⁴ M, vorliegt, oder
wobei die Zusammensetzung insbesondere einen Hilfsstoff, ein Verdünnungsmittel oder ein Lösungsmittel umfasst, wobei die Zusammensetzung so formuliert ist, dass sie eine Umhüllung bildet.

11. Zusammensetzung, umfassend in Kombination eine Menge an Saatgut einer Pflanze und eine Menge eines synthetischen Peptids, dessen Sequenz eine Sequenz umfasst oder aus einer Sequenz besteht, die mit der eines natürlich in der Pflanze vorkommenden miPEP identisch ist,
wobei das synthetische Peptid eine Sequenz aufweist, die eine Sequenz umfasst oder aus einer Sequenz besteht, die mit der des miPEPP172c identisch ist, wobei das miPEPP172c eine Aminosäuresequenz besitzt, die mindestens 80 % Identität mit der Sequenz SEQ ID NO: 2 aufweist,
wobei das miPEP172c insbesondere eine Aminosäuresequenz besitzt, die aus der Sequenz SEQ ID NO: 2 besteht,
wobei die Zusammensetzung insbesondere so formuliert ist, dass sie einen umhüllten Samen bildet.

12. Saatgut, das mit einer Zusammensetzung umhüllt ist, die ein miPEP umfasst, das in der Lage ist, die Akkumulation einer miR in der aus dem Saatgut hervorgegangenen Pflanze zu modulieren, wobei die miR die Expression mindestens eines Gens reguliert, das an der Nodulation in der Pflanze beteiligt ist,
wobei das miPEP das miPEP172c ist, wobei das miPEP172c eine Aminosäuresequenz besitzt, die mindestens 80 % Identität mit der Sequenz SEQ ID NO: 2 aufweist,
wobei das miPEP172c insbesondere eine Aminosäuresequenz besitzt, die aus der Sequenz SEQ ID NO: 2 besteht.

13. Verwendung eines miPEP zur Kultivierung von Bakterien, insbesondere von Bakterien der Familie der *Rhizobiaceae,* wobei die Verwendung einen Schritt umfasst, bei dem die Bakterien mit Folgendem in Kontakt gebracht werden:
- einer Mischung, umfassend eine Pflanze oder einen Pflanzenteil, insbesondere eine Wurzelkultur, und ein Peptid, dessen Sequenz eine Sequenz umfasst oder aus einer Sequenz besteht, die mit der eines in der Pflanze natürlich vorkommenden miPEP identisch ist, wobei das natürlich vorkommende miPEP ein Peptid ist, dessen Sequenz von einem offenen Leserahmen kodiert wird, der sich in 5' auf dem primären Transkript einer miR befindet, wobei das miPEP in der Lage ist, die Akkumulation der miR zu modulieren, wobei die miR die Expression mindestens eines Gens reguliert, das an der Nodulation in der Pflanze beteiligt ist, oder
- mit einer transgenen Pflanze, wie oben definiert,
wobei die Pflanze, der Pflanzenteil und die transgene Pflanze zur Nodulation mit dem Bakterium befähigt sind,
wobei das miPEP das miPEP172c ist, wobei das miPEP172c eine Aminosäuresequenz besitzt, die mindestens 80 % Identität mit der Sequenz SEQ ID NO: 2 aufweist,
wobei das miPEP172c insbesondere eine Aminosäuresequenz besitzt, die aus der Sequenz SEQ ID NO: 2 besteht.

14. Verwendung eines miPEP zur Herstellung von Bakterieninokulum, insbesondere von Bakterieninokulum der Familie der *Rhizobiaceae,* wobei die Verwendung Folgendes umfasst:
- einen Schritt zum Co-Kultivieren von Bakterien mit einem lebenden Pflanzenmaterial, einer sogenannten Wirtspflanze, die zumindest teilweise einem konstitutiven Teil der Wurzel einer Pflanze entspricht, die mit den Bakterien eine Nodulation bilden kann, und
- einen Schritt zum Inkontaktbringen einer Menge eines Peptids mit der oben genannten Co-Kultur,
wobei das Peptid eine Sequenz aufweist, die eine Sequenz umfasst oder aus einer Sequenz besteht, die mit der des natürlich in der Pflanze vorkommenden miPEP identisch ist, wobei das natürlich vorkommende miPEP ein Peptid ist, dessen Sequenz von einem offenen Leserahmen kodiert wird, der sich in 5' auf dem primären Transkript einer miR befindet, wobei das miPEP in der Lage ist, die Akkumulation der miR zu modulieren, wobei die miR die Expression von mindestens einem Gen reguliert, das an der Nodulation in der Pflanze beteiligt ist,
wobei das miPEP das miPEP172c ist, wobei das miPEP172c eine Aminosäuresequenz besitzt, die mindestens 80 % Identität mit der Sequenz SEQ ID NO: 2 aufweist,
wobei das miPEP172c insbesondere eine Aminosäuresequenz besitzt, die aus der Sequenz SEQ ID NO: 2 besteht.

15. Bakterieninokulum, insbesondere ein Bakterieninokulum der Familie der *Rhizobiaceae,* das zur Inokulation einer Wirtspflanze geeignet ist, umfassend mindestens:
eine Bakterie und ein Peptid, dessen Sequenz eine Sequenz umfasst oder aus einer Sequenz besteht, die mit der eines miPEP identisch ist, das natürlich in der Wirtspflanze vorkommt,
wobei das in der Wirtspflanze natürlich vorkommende miPEP ein Peptid ist, dessen Sequenz von einem offenen Leserahmen kodiert wird, der sich in 5' auf dem primären Transkript einer miR befindet, wobei das miPEP in der Lage ist, die Akkumulation der miR zu modulieren, wobei die miR die Expression von mindestens einem Gen reguliert, das an der Nodulation in der Wirtspflanze beteiligt ist,
wobei das miPEP das miPEP172c ist, wobei das miPEP172c eine Aminosäuresequenz besitzt, die mindestens 80 % Identität mit der Sequenz SEQ ID NO: 2 aufweist,
wobei das miPEP172c insbesondere eine Aminosäuresequenz besitzt, die aus der Sequenz SEQ ID NO: 2 besteht.

## Claims

1. Use of a miPEP exogenously introduced into a plant for promoting nodulation, between said plant and a bacterium, said miPEP being also naturally present in said plant,
said exogenously introduced miPEP being either a peptide produced outside the plant or a peptide produced in the plant following the non-natural introduction of a nucleic acid encoding said miPEP into said plant,
said exogenously introduced miPEP being a peptide having an amino acid sequence comprising or consisting of a sequence identical to that of a miPEP naturally present in said plant,
said miPEP naturally present in said plant being a peptide of from 3 to 100 amino acids the sequence of which is coded by an open reading frame at the 5' portion of a primary transcript of a miR,
said miPEP being capable of modulating the accumulation of said miR in said plant, which miR regulates the expression of at least one gene involved in the nodulation in said plant,
the sum of the amount of said exogenously introduced miPEP and of the amount of said naturally present miPEP being strictly greater than the amount of said naturally present miPEP,
said miPEP being the miPEP172c having an amino acid sequence having at least 80% identity with the amino acid sequence SEQ ID NO: 2,
in particular said miPEP172c having an amino acid sequence consisting of SEQ ID NO: 2, preferably, said miR being the miR172c having in particular a nucleotide sequence consisting of SEQ ID NO: 1.

2. Use according to claim 1, wherein said plant is:
- a leguminous plant, such as lotus (*Lotus sp*.), soybean (*Glycine max*), peanut (*Arachis hypogaea*), common bean (*Phaseolus vulgaris*), pea (*Pisum sativum*), lentil (*Lens culinaris*), chickpea (*Cicer arietinum*), broad bean and field bean (*Vicia faba*), vetches (*Vicia* sp.), vetchlings (*Lathyrus* sp.), alfalfa (*Medicago* sp.), clover (*Trifolium* sp.), lupin (*Lupinus* sp.), mungo bean (*Vigna radiata*), liquorice (*Glycyrrhiza glabra*), rosewood (*Dalbergia*)*,* trefoil (*Lotus corniculatus),* sainfoin (*Onobrychis viciifolia)*, rooibos *(Aspalathus linearis),* or fenugreek (*Trigonella foenum-graecum)*, or
- a sugar beet (Beta vulgaris).

3. Use according to any one of the preceding claims, wherein said bacterium is a bacterium from the *Rhizobiaceae* family, in particular selected from the genera *Rhizobium, Sinorhizobium, Mesorhizobium, Bradyrhizobium* or *Azorhizobium.*

4. Method for promoting the nodulation between a plant and a bacterium, comprising a step of introducing a miPEP exogenously into a plant, said miPEP being also present naturally in said plant,
said exogenously introduced miPEP being a peptide having an amino acid sequence comprising or consisting of a sequence identical to that of a miPEP naturally present in said plant,
said exogenously introduced miPEP being a peptide of from 3 to 100 amino acids the sequence of which comprises or consists of a sequence identical to that of said naturally present miPEP, which sequence of the naturally present miPEP is coded by an open reading frame at the 5' portion of the primary transcript of a miR, said miPEP being capable of modulating the accumulation of said miR, which miR regulates the expression of at least one gene involved in the nodulation in said plant,
the sum of the amount of said exogenously introduced miPEP and of the amount of said naturally present miPEP being strictly greater than the amount of said naturally present miPEP,
said miPEP being the miPEP172c having an amino acid sequence having at least 80% identity with the sequence SEQ ID NO: 2, in particular said miPEP172c having an amino acid sequence consisting of the sequence SEQ ID NO: 2,
preferably, said miR being miR172c having in particular a nucleotide sequence consisting of SEQ ID NO: 1,
said bacterium being in particular of the *Rhizobiaceae* family,
said plant being in particular a leguminous plant.

5. Method according to claim 4, for promoting nodulation between a *Glycine max* plant and a *Bradirhizobium japonicum* bacterium, wherein miPEP172c is exogenously introduced into said *Glycine max* plant, said miPEP172c being naturally present in said *Glycine max* plant,
said miPEP172c exogenously introduced being a peptide comprising or consisting of a sequence identical to that of said naturally present miPEP172c, which naturally present miPEP172c is a peptide of from 3 to 100 amino acids of which the sequence is coded by an open reading frame at the 5' portion of the primary transcript of the miR172c,
said miPEP172c being capable of increasing the accumulation of said miR172c, which miR172c regulates the expression of at least one gene involved in the nodulation in *Glycine max,*
the sum of the amount of said miPEP172c exogenously introduced and of the amount of said naturally present miPEP172c being strictly greater than the amount of said naturally present miPEP172c.

6. Method according to claim 4 or 5, wherein said miPEP is administered to the plant:
- externally, preferably by watering, by spraying, or by the addition of a fertiliser, a soil, a culture substrate, or with the aid of a support in contact with the plant, said miPEP being in particular administered to the plant in the form of a composition comprising from 10⁻⁹ M to 10⁻⁴ M of said miPEP, especially 10⁻⁹, 10⁻⁸, 10⁻⁷, 10⁻⁶, 10⁻⁵ or 10⁻⁴ M of said miPEP,
- externally, preferably by watering, by spraying, or by the addition of a fertiliser, a soil, a culture substrate, or with the aid of a support in contact with the grain or the seed, said miPEP being in particular administered to the grain or the seed in the form of a composition comprising from 10⁻⁹ M to 10⁻⁴ M of said miPEP, especially 10⁻⁹, 10⁻⁸, 10⁻⁷, 10⁻⁶, 10⁻⁵ or 10⁻⁴ M of said miPEP, or
- by way of a nucleic acid coding said miPEP, said nucleic acid being introduced into the plant, but said nucleic acid not comprising the complete sequence of the corresponding miR.

7. Method for producing a transgenic plant, said method comprising:
**a)** a step of introducing a nucleic acid coding a miPEP having from 3 to 100 amino acids into a plant, but said nucleic acid not comprising the complete sequence of the corresponding miR, or into at least a cell of said plant, in conditions allowing the expression of said miPEP,
said miPEP also being present naturally in said plant, said naturally present miPEP being a peptide of which the sequence is coded by an open reading frame at the 5' portion of the primary transcript of a miR, said miPEP being capable of modulating the accumulation of said miR in the plant, which miR regulates the expression of at least one gene involved in the nodulation, and
**b)** a step of culturing the plant, or at least a cell of said plant, obtained in step a) in conditions making it possible to obtain a transgenic plant,
said miPEP being the miPEP172c having an amino acid sequence having at least 80% identity with the amino acid sequence SEQ ID NO: 2,
in particular said miPEP172c having an amino acid sequence consisting of SEQ ID NO: 2, preferably, said miR being the miR172c having in particular a nucleotide sequence consisting of SEQ ID NO: 1.

8. Transgenic plant such as obtained by the method according to claim 7.

9. Peptide having an amino acid sequence having at least 80% identity, preferably at least 90% identity, with the amino acid sequence SEQ ID NO: 2,
preferably said peptide comprising or consisting of sequence SEQ ID NO: 2,
said peptide being capable of modulating the accumulation of miR172c.

10. Composition comprising:
- miPEP172c as an active substance, said miPEP172c comprising or consisting of an amino acid sequence having at least 80% identity with the sequence SEQ ID NO: 2,
in particular said miPEP172c comprising or consisting of an amino acid sequence consisting of SEQ ID NO: 2,
said miPEP172c being in particular at a concentration from 10-9 M to 10-4 M of said miPEP, especially 10-9, 10-8, 10-7, 10-6, 10-5 or 10-4 M, or
in particular, said composition further comprising an excipient, a diluent, or a solvent,
in particular, said composition being formulated so as to form a coated product.

11. Composition comprising, in combination, a quantity of seeds of a plant and a quantity of a synthetic peptide of which the sequence comprises or consists of a sequence identical to that of a miPEP naturally present in said plant,
said synthetic peptide having a sequence comprising or consisting of a sequence identical to that of miPEP172c, said miPEP172c having an amino acid sequence showing at least 80% identity with the sequence SEQ ID NO: 2,
in particular said miPEP172c having an amino acid sequence consisting of SEQ ID NO: 2, in particular, said composition being formulated so as to form a coated seed.

12. Coated seed with a composition comprising a miPEP capable of modulating the accumulation of a miR in the plant produced from the seed, which miR regulates the expression of at least one gene involved in the nodulation in said plant,
said miPEP being the miPEP172c having an amino acid sequence having at least 80% identity with the amino acid sequence SEQ ID NO: 2,
in particular said miPEP172c having an amino acid sequence comprising or consisting of SEQ ID NO: 2.

13. Use of a miPEP for culturing bacteria, in particular said bacterium being of the *Rhizobiaceae* family, said use comprising a step of contacting said bacteria with:
- a mixture comprising a plant or a plant part, especially a root culture, and a peptide of which the sequence comprises or consists of a sequence identical to that of a miPEP naturally present in said plant, said naturally present miPEP being a peptide, the sequence of which is coded by an open reading frame at the 5' portion of the primary transcript of a miR, said miPEP being capable of modulating the accumulation of said miR, which miR regulates the expression of at least one gene involved in the nodulation in said plant, or
- a transgenic plant as defined above,
the plant, the plant part, and the transgenic plant being able to form a symbiosis with said bacterium,
said miPEP being the miPEP172c having an amino acid sequence having at least 80% identity with the sequence SEQ ID NO: 2,
in particular said miPEP172c having an amino acid sequence consisting of the sequence SEQ ID NO: 2.

14. Use of a miPEP for producing a bacterial inoculum, in particular an inoculum of bacteria from the *Rhizobiaceae* family, said use comprising:
- a step in which bacteria are co-cultured with a living plant material, referred to as the host plant, corresponding at least in part to a constituent part of the root of a plant capable of forming a nodulation with said bacteria, et
- a step of contacting a quantity of a peptide with said co-culture,
said peptide having a sequence comprising or consisting of a sequence identical to that of a miPEP naturally present in said host plant, said naturally present miPEP being a peptide, the sequence of which is coded by an open reading frame at the 5' portion of the primary transcript of a miR, said miPEP being capable of modulating the accumulation of said miR, which miR regulates the expression of at least one gene involved in the nodulation in said host plant,
said miPEP being the miPEP172c having an amino acid sequence having at least 80% identity with the amino acid sequence SEQ ID NO: 2,
in particular said miPEP172c having an amino acid sequence consisting of SEQ ID NO: 2.

15. Bacterial inoculum, in particular an inoculum of bacteria from the *Rhizobiaceae* family, suitable for the inoculation of a host plant, comprising at least one bacterium and a peptide, the sequence of which comprises or consists of a sequence identical to that of a miPEP naturally present in the host plant,
said miPEP naturally present in the host plant being a peptide, the sequence of which is coded by an open reading frame at the 5' portion of the primary transcript of a miR, said miPEP being capable of modulating the accumulation of said miR, which miR regulates the expression of at least one gene involved in the nodulation in said host plant,
said miPEP being the miPEP172c having an amino acid sequence having at least 80% identity with the amino acid sequence SEQ ID NO: 2,
in particular said miPEP172c having an amino acid sequence consisting of SEQ ID NO: 2.
